# FASCICULE DE BREVET EUROPEEN

(11) **EP 4 153 305 B1**
(45) Date de publication et mention de la délivrance du brevet: **30.04.2025**
(21) Numéro de dépôt: 21728062.7
(22) Date de dépôt: 21.05.2021
(51) Int. Cl.: A61P 17/00, A23L 33/105, A61K 8/9789, A61K 36/185, A61Q 19/00

(54) **HYDROLYSAT DE PROTÉINES DU TOURTEAU DES GRAINES DE MORINGA PEREGRINA POUR SON APPLICATION EN TANT QUE MÉDICAMENT, SON PROCÉDÉ D'OBTENTION ET COMPOSITIONS PHARMACEUTIQUES ET DERMATOLOGIQUES**
PROTEINHYDROLYSAT AUS MORINGA PEREGRINA-SAMENKUCHEN ZUR ANWENDUNG ALS MEDIKAMENT, VERFAHREN ZU SEINER HERSTELLUNG UND PHARMAZEUTISCHE UND DERMATOLOGISCHE ZUSAMMENSETZUNGEN
PROTEIN HYDROLYSATE OF MORINGA PEREGRINA SEED CAKE FOR ITS APPLICATION AS A MEDICAMENT, PROCESS FOR OBTAINING SAME AND PHARMACEUTICAL AND DERMATOLOGICAL COMPOSITIONS

(30) Priorité: 21.05.2020 FR 2005426
(43) Date de publication de la demande: 29.03.2023
(73) Titulaire: ALULA PEREGRINA TRADING, 43514 AlUla city (SA)
(72) Inventeur: DODINET, Elizabeth, 12560 Saint-Laurent d'Olt (FR); BOURGETEAU, Vincent, 56130 Férel (FR)
(74) Mandataire: Loyer & Abello
(86) Numéro de dépôt international: PCT/EP2021/063704
(87) Numéro de publication internationale: WO 2021/234165

(56) Documents cités:
- CN-A- 107 012 190
- FR-A1- 2 776 519
- BAGNATO A ET AL: "Role of the endothelin axis and its antagonists in the treatment of cancer", BRITISH JOURNAL OF PHARMACOLOGY, WILEY-BLACKWELL, UK, vol. 163, no. 2, 18 April 2011 (2011-04-18), pages 220 - 233, XP071170825, ISSN: 0007-1188, DOI: 10.1111/J.1476-5381.2011.01217.X
- A. P. DE GROOT ET AL: "Effects of Severe Alkali Treatment of Proteins on Amino Acid Composition and Nutritive Value", THE JOURNAL OF NUTRITION, vol. 98, no. 1, 1 May 1969 (1969-05-01), US, pages 45 - 56, XP055753138, ISSN: 0022-3166, DOI: 10.1093/jn/98.1.45
- SHAY JERRY W. ET AL: "Senescence and immortalization: role of telomeres and telomerase", CARCINOGENESIS, vol. 26, no. 5, 1 May 2005 (2005-05-01), GB, pages 867 - 874, XP055856322, ISSN: 0143-3334, Retrieved from the Internet <URL:https://academic.oup.com/carcin/article-pdf/26/5/867/7088814/bgh296.pdf> DOI: 10.1093/carcin/bgh296
- YOON SOMY ET AL: "HDAC Inhibitors: Therapeutic Potential in Fibrosis-Associated Human Diseases", INTERNATIONAL JOURNAL OF MOLECULAR SCIENCES, vol. 20, no. 6, 16 March 2019 (2019-03-16), pages 1329, XP055958567, DOI: 10.3390/ijms20061329
- RIBERO S . ET AL: "Skin phenotypes can offer some insight about the association between telomere length and cancer susceptibility", MEDICAL HYPOTHESES, vol. 97, 1 December 2016 (2016-12-01), US, pages 7 - 10, XP055958566, ISSN: 0306-9877, Retrieved from the Internet <URL:https://www.sciencedirect.com/science/article/pii/S0306987716305187/pdfft?md5=c6b705b01ac8c33edc5528b4f2113876&pid=1-s2.0-S0306987716305187-main.pdf> DOI: 10.1016/j.mehy.2016.10.010
- CARRIE WATERMAN ET AL: "Stable, water extractable isothiocyanates from Moringa oleifera leaves attenuate inflammation in vitro", PHYTOCHEMISTRY, vol. 103, 1 July 2014 (2014-07-01), Amsterdam , NL, pages 114 - 122, XP055321973, ISSN: 0031-9422, DOI: 10.1016/j.phytochem.2014.03.028
- SÖRENSEN-ZENDER INGA ET AL: "Zinc- [alpha] 2-Glycoprotein Exerts Antifibrotic Effects in Kidney and Heart", JOURNAL OF THE AMERICAN SOCIETY OF NEPHROLOGY, vol. 26, no. 11, 1 November 2015 (2015-11-01), US, pages 2659 - 2668, XP055958562, ISSN: 1046-6673, Retrieved from the Internet <URL:https://jasn.asnjournals.org/content/jnephrol/26/11/2659.full.pdf?with-ds=yes> DOI: 10.1681/ASN.2014050485
- H M ABU-TARBOUSH ET AL: "Characterization of Hydrolysates Produced by Enzymatic hydrolysis of Camel Casein and Protein Isolates of Al-Ban (Moringa peregrina) and Karkade (Hibiscus sabderiffa) Seeds", J. SAUDI SOCFOR AGRIC. SCI., vol. 4, no. 2, 1 January 2005 (2005-01-01), pages 61 - 82, XP055753092
- NASHEF A S ET AL: "EFFECTS OF ALKALI ON PROTEINS. DISULFIDES AND THEIR PRODUCTS", JOURNAL OF AGRICULTURAL AND FOOD CHEMISTRY, AMERICAN CHEMICAL SOCIETY, BOOKS AND JOURNALS DIVISION|, vol. 25, no. 2, 1 March 1977 (1977-03-01), pages 245 - 251, XP001087830, ISSN: 0021-8561, DOI: 10.1021/JF60210A020
- RAO M B ET AL: "Molecular and biotechnological aspects of microbial proteases", MICROBIOLOGY AND MOLECULAR BIOLOGY REVIEWS, AMERICAN SOCIETY FOR MICROBIOLOGY, US, vol. 62, no. 3, 1 September 1998 (1998-09-01), pages 597 - 635, XP002245348, ISSN: 1092-2172
- "Co? and Post-Translational Modifications of Therapeutic Antibodies and Proteins", 2 April 2019, JOHN WILEY & SONS, INC, Hoboken, NJ, USA, ISBN: 978-1-119-05331-6, article T. SHANTHA RAJU: "Proteolysis of Proteins", pages: 183 - 202, XP055753173, DOI: 10.1002/9781119053354.ch15
- YOUNG-SHICK HONG ET AL: "Molecular Weight Distribution of Protein Hydrolysate by the Enzymic Hydrolysis of Weakly Acid-Treated Wheat Gluten", FOOD SCIENCE AND TECHNOLOGY RESEARCH, vol. 7, no. 2, 1 January 2001 (2001-01-01), CH, pages 126 - 130, XP055753367, ISSN: 1344-6606, DOI: 10.3136/fstr.7.126
- ZHOU XU ET AL: "Purification and identification immunomodulatory peptide from rice protein hydrolysates", FOOD AND AGRICULTURAL IMMUNOLOGY., vol. 30, no. 1, 1 January 2019 (2019-01-01), GB, pages 150 - 162, XP055753384, ISSN: 0954-0105, DOI: 10.1080/09540105.2018.1553938
- MENDEL FRIEDMAN ET AL: "Protein-Alkali Reactions: Chemistry, Toxicology, and Nutritional Consequences", 1 January 1984 (1984-01-01), XP009524239, ISBN: 978-1-4684-4790-3, Retrieved from the Internet <URL:https://link.springer.com/chapter/10.1007/978-1-4684-4790-3_18>
- KJAER A ET AL: "ISOTHIOCYANATES IN MYROSINASE-TREATED SEED EXTRACTS OF MORINGA PEREGRINA", PHYTOCHEMISTRY, ELSEVIER, AMSTERDAM, NL, vol. 18, no. 9, 1 January 1979 (1979-01-01), pages 1485 - 1487, XP009024211, ISSN: 0031-9422, DOI: 10.1016/S0031-9422(00)98480-2
- AFSHARYPUORA S ET AL: "Volatile constituents of the seed kernel and leaf of Moringa peregrina (forssk.) Fiori, Agricolt. cultivated in Chabahar (IRAN)", IRANIAN JOURNAL OF PHARMACEUTICAL SCIENCES, IRANIAN SOCIETY OF PHARMACEUTICAL SCIENTISTS, IR, vol. 6, no. 2, 1 January 2010 (2010-01-01), pages 141 - 144, XP009508062, ISSN: 1735-2444
- AL-DABBAS MAHER M ET AL: "Chemical composition and oil components in seeds of Moringa peregrina (Forssk) Fiori", CROP RESEARCH, AGRICULTURAL RESEARCH INFORMATION CENTRE, HISAR, IN, vol. 40, 1 January 2010 (2010-01-01), pages 161 - 167, XP009163458, ISSN: 0970-4884

## Description

### Domaine technique

L'invention se rapporte au domaine de la pharmacie et la dermatologie et plus particulièrement celui des ingrédients actifs entrant dans la formulation des médicaments. L'invention a trait à un procédé d'obtention d'un hydrolysat de protéines du tourteau des graines de *Moringa peregrina.* L'invention concerne également l'hydrolysat de protéines du tourteau des graines de *Moringa peregrina,* les compositions pharmaceutiques et dermatologiques comprenant un tel hydrolysat et destinées à être utilisées pour le traitement de maladies fibrotiques, le traitement de l'inflammation, du cancer, de maladies infectieuses de type virales.

### Arrière-plan technologique

Les Moringaceae constituent une famille mono-générique (un seul genre, *Moringa* Adans), élément de la flore Saharo-sindienne, constituée d'entre douze et quatorze espèces selon les auteurs, réparties de l'Afrique orientale à l'Asie. Le genre est classiquement divisé en 3 sections qui ne sont, cependant, pas confirmées comme monophylétiques par les analyses phylogénétiques. Ces dernières ont plutôt mis en évidence des clades axés sur certains caractères morphologiques : pachycaules (« bottle trees ») ; tubéreux (« tuberous trees ») et ni pachycaules - ni tubéreux (en anglais « slender trees »). L'espèce *Moringa peregrina* (Forssk.) Fiori, appartient au troisième groupe. Les quelques études génétiques sur le genre ou la famille confirment la réalité de l'espèce par rapport aux autres espèces dans le genre, notamment vis-à-vis du Moringa indien, *Moringa oleifera* Lam. (voir notamment les articles: OLSON, M. E., 2002, Combining Data from DNA Sequences and Morphology for a Phylogeny of Moringaceae (Brassicales), Systematic Botany 27(1): p. 55-73; HASSANEIN, A. M. A. et al., 2018, Morphological and genetic diversity of Moringa oleifera and Moringa peregrina genotypes, Horticulture, Environment and Biotechnology 59(2): p. 251-261). Un article récent sur le *Moringa peregrina* échantillonné sur différentes localités en Arabie Saoudite a conclu, en utilisant des marqueurs ITS, à une stabilité génétique de l'espèce (ALAKLABI, A., 2015, Genetic diversity of Moringa peregrina species in Saudi Arabia with ITS sequences, Saudi Journal of Biological Sciences 22: p. 186-190) avec cependant un haut niveau de variation génétique intra-populations.

L'espèce *Moringa peregrina* se retrouve dans les environnements rocheux du Yémen, d'Oman, et d'Arabie Saoudite, en Afrique orientale, au Soudan, en Éthiopie, Érythrée, Somalie et Djibouti. Sa présence en Iran semble limitée aux provinces sud-orientales mais demande à être confirmée (PROTA14 = MUNYANZIZA, E. et al. Vegetable oils/Oléagineux, Moringa peregrina (Forssk.) Fiori, http://database.prota.org/protahtml/moringa peregrina_fr.htm, accès le 23/10/2019). Au Levant et en Égypte, l'espèce ne représente, aujourd'hui, plus que de rares stations dispersées, relictuelles (à l'exception de quelques peuplements altitudinaux), principalement dans les secteurs de l'aire soudanienne. *Moringa peregrina* est également considéré aujourd'hui comme rare et en danger au Soudan et au Yémen. *Moringa peregrina* occupe par rapport aux autres espèces de son clade les habitats les plus arides et les plus inhospitaliers. Il est apparemment plus résistant à la sécheresse que *Moringa oleifera* qui est planté commercialement à grande échelle dans les zones tropicales et subtropicales. Des travaux récents ont montré que la taille et la grosseur des graines impactait favorablement le temps de germination et le taux et la rapidité de la croissance des jeunes individus (GOMAA, N. H. et al., 2011, Seed germination, seedling traits, and seed bank of the tree Moringa peregrina (Moringaceae) in a hyper-arid environment, American Journal of Botany 98(6): p. 1024-1030), indiquant un ajustement dans l'allocation des ressources sur la qualité de la graine plutôt que le nombre, ce qui permet à *Moringa peregrina* de se reproduire efficacement dans des environnements abiotiques extrêmes (hyperarides). Les graines de *Moringa peregrina* ont une mesotesta centrale plus épaisse, en termes de couche de cellules, que celles de *Moringa oleifera.*

Il existe quelques mentions historiques qui tendent à indiquer que l'huile de *Moringa peregrina* faisait l'objet d'un commerce actif dans les débuts de l'Islam dans la région d'AlUla (NASEEF, A. A. S., 1995, *Al-'Ul* 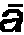 *, A study of Cultural and Social Heritage*)*.* L'huile produite à partir de *Moringa peregrina* est aujourd'hui destinée principalement à l'autoconsommation ou aux marchés locaux. En Arabie Saoudite, les feuilles étaient utilisées traditionnellement en décoction en usage interne pour soigner le diabète, les maladies du colon, les maladies des yeux et les anémies (ABDEL-KADER et al., A survey on the traditional plants used in AI Kobah village, Saudi Pharmaceutical Journal 26(6): p. 817-821) et comme diurétique, rubéfiant et astringent (AQEEL, A. A. M. et al., 1984, Plants used in Arabia Folk medicine, Report submitted to Saudi Arabian National Centre for Science and Technology, Riyadh, Saudi Arabia). En Oman, l'huile, extraite par les femmes à la fin de l'été, est utilisée contre les migraines, la fièvre, les brûlures, les lacérations et les fractures, la constipation et les douleurs d'estomac, contre les douleurs musculaires et contre la sécheresse capillaire (GHAZANFAR, S. A., 1994, Handbook of Arabian Medicinal Plants, 1e éd., CRC Press, Boca Raton, Ann Harbor, s.u. ; GHAZANFAR, S.A., 1998, Plants of Economic Importance, cap. 15, in GHAZANFAR, S.A. et al. (éd.) Vegetation of the Arabian Peninsula. Geobotany 25, p. 241-264, Kluwer Academic Publishers, tableau 11.1, p. 247 et 11.7 p. 251). Elle était également utilisée dans des compositions parfumées (GHAZANFAR, S. A., 1998, p. 259) et en Oman et au Yémen comme lotion faciale (GHAZANFAR, S. A. et al., 1996, Two multi-purpose seed oils form Oman. Plants for Food and Medicine. Paper presented at the joint meeting of the Society for Economic Botany and International Society for Ethnopharmacology, 1-7 July 1996, London).

On connait un certain nombre d'extraits provenant de la graine de *Moringa oleifera* utiles plus particulièrement dans le domaine cosmétique. Dans le domaine dermatologique, on connaît du document FR2776519 que des extraits protéiques de graines de *Moringa oleifera,* connus pour leurs effets clarifiants sur les eaux turbides, présentent sur la peau et les muqueuses un effet adoucissant, conditionneur physiologique, hydratant, restructurant, réparateur et un effet antipollution. Dans ce document les principes actifs sont des protéines de poids moléculaires compris entre 6 500 et 8 800 Da qui sont obtenues par extraction aqueuse sur le tourteau de *Moringa oleifera.*

Dans le domaine pharmaceutique, on connaît du document KR20140143655 une composition contenant un extrait hydrosoluble de feuilles de *Moringa oleifera* comme ingrédient actif pour traiter ou prévenir le cancer. Le procédé d'hydrolyse enzymatique sur des extraits de feuilles de *Moringa oleifera* permet d'isoler des protéines de poids moléculaires compris entre 6 000 et 8 000 Da.

Le document CN107012190 concerne l'utilisation des graines de *Moringa oleifera* pour obtenir, après avoir extrait l'huile et effectué une hydrolyse enzymatique en utilisant les rayonnements à micro-ondes, des polypeptides des protéines de la graine. Le produit obtenu est utilisé par voie orale et est utile pour une activité anticancer. Ce produit sous forme de poudre est décrit comme ayant en fait une action pour réduire les effets secondaires de la chimiothérapie et pour augmenter l'appétit de patients présentant un cancer du foie, ou pour contrôler l'index des leucocytes et la température corporelle.

Enfin, on connaît du document IN2009CH02906 qu'un extrait aqueux du tourteau de *Moringa oleifera* comprenant du glucosinolate combiné à des protéines cationiques peut être utilisé comme produit anti-diabétique.

Tous les documents précités concernent l'utilisation de l'espèce *Moringa oleifera ;* aucun ne décrit l'utilisation dans le domaine pharmaceutique ou dermatologique d'extraits provenant de l'espèce *Moringa peregrina.*

On connait également le document de ABU TARBOSH *et al.* XP055753092, 2005 qui décrit l'extraction d'un hydrolysat à partir de graines de *Moringa peregrina* décortiquées produit par hydrolyse enzymatique pendant une durée de 10 heures. Le but de cette extraction est d'obtenir un produit présentant une haute capacité d'absorption de l'huile et de l'eau.

Un extrait de l'huile de *Moringa peregrina* réalisé à partir de graines d'Egypte, avec un mélange dichlorométhane/ méthanol (1/1) a montré une activité sur trois lignées de cellules cancéreuses humaines MCF-7 (adénocarcinome du sein), Hep-G2 (carcinome hépatocellulaire) et HCT-116 (carcinome du colon) avec des valeurs IC50 de 2,92, 9,40, et 9,48 µg/mn (ABD EL BAKY et al., 2013, Characterization of Egyptian Moringa peregrina seed oil and its bioactivities, International Journal of Management Sciences and Business Research, 2(7): p. 98-108). Les auteurs ont également mis en évidence une importante activité anti-oxydante par essais *DPPH* (2,2-diphényl 1-picrylhydrazyle), *ABTS* (anion-scavenging capability and reducing power) et anti-proliférative. Du fait de procédés d'obtentions de l'extrait différents de ceux de l'invention, les molécules obtenues sont à polarité très différentes.

ABOU-HASHEM et collègues (ABOU-HASHEM, M. M. M. et al., 2019, Induction of sub-G0 arrest and apoptosis by seed extract of Moringa peregrina (Forssk.) Fiori in cervical and prostate cancer cell lines, Journal of Integrative Medicine 17 : p. 410-422) ont également mis en évidence une activité d'induction de l'arrêt sub-Go et d'apostose sur les lignées cellulaires cancéreuses cervicales (HELA) et de la prostate (PC-3). Le protocole impliquait des graines de *Moringa peregrina* égyptiennes, réduites en poudre et extraites à 95% d'éthanol, puis dissoutes dans une solution aqueuse à partir desquelles trois extraits fractionnés étaient étudiés (fractions par éther de pétrole (PE), CHCl3 et EtOAc) ainsi qu'un extrait hydrolique résidu de l'extraction. L'activité a été mise en évidence pour la fraction totale chloroforme et a été attribuée aux acides gras saturés et insaturés et aux polyphénols sans étude spécifique de la mécanistique. Du fait de procédés d'obtention de l'extrait différents de ceux de la présente invention, les molécules actives obtenues sont à polarité très différentes.
Carrie Waterman et al. (« Stable, water extractable isothiocyanates from Moringa oleifera leaves attenuate inflammation in vitro », Phytochemistry, vol. 103, 1 juillet 2014, pages 114-122) divulgue la préparation d'un extrait des feuilles de *Moringa oleifera* riche en composés isothiocyanates obtenu par extraction à l'eau ou à l'éthanol. L'extrait obtenu démontre un effet antiinflammatoire *in vitro.*

Considérant ce qui précède, un problème que se propose de résoudre l'invention est de développer des produits nouveaux à base d'un extrait de l'espèce *Moringa peregrina* du genre *Moringa* et de la famille Moringaceae qui soient utilisables en pharmacie ou dermatologie et faciles à mettre en œuvre.

Ainsi, la Demanderesse a de manière surprenante mis en évidence un hydrolysat de protéines particulier obtenu du tourteau des graines de *Moringa peregrina* pour son application en tant que médicament, et notamment pour le traitement de maladies fibrotiques (en tant qu'inhibiteur de la furine convertase), le traitement de l'inflammation, du cancer, de maladies infectieuses de type virales.

L'espèce *Moringa peregrina* pousse dans des climats très arides. Ainsi, sa faculté à résister à la sécheresse et à se reproduire dans des conditions extrémophiles lui a permis d'acquérir des caractéristiques particulières uniques, que la Demanderesse a pu identifier par la mise en oeuvre d'un procédé d'extraction spécifique sur le tourteau des graines de *Moringa peregrina.* L'hydrolysat de protéines selon l'invention s'avère posséder des propriétés en tant que médicament et il est démontré qu'il présente notamment une activité inhibitrice très élevée de la furine convertase.

La furine convertase (ci-après nommée furine) est une protéine transmembranaire de type 1 avec 794 acides aminés exprimés dans différents types cellulaires. La furine est une protéine qui a montré être impliquée dans un grand nombre de processus biologiques (BRAUN E. et al., 2019, Furin-mediated protein processing in infectious diseases and cancer, Clinical & Translational Immunology https://doi.org/10.1002/cti2.1073). Elle intervient notamment dans la cicatrisation des plaies ainsi que dans la régulation de la fibrose dans le traitement des affections dans lesquelles la fibrose est un mécanisme majeur de réparation tissulaire ou lorsqu'une fibrose excessive entraîne un dérangement pathologique et un dysfonctionnement du tissu (voir à ce propos le document WO200409113 qui concerne l'utilisation de convertase pour réduire la cicatrisation lors de guérison d'une blessure et pour réduire la fibrose dans le traitement d'états fibrotiques). La cicatrisation des plaies chez l'adulte est un processus de réparation complexe. Les plaies peuvent entraîner des dommages, des blessures ou des traumatismes à un tissu ou un organe interne comme les poumons, les reins, le coeur, les intestins, les tendons ou le foie. Le processus de guérison des plaies sur un tissu commence généralement par une réponse hémostatique déclenchée par des dommages aux vaisseaux sanguins de la peau. Au cours de ce processus, le tissu conjonctif qui se forme pendant le processus de guérison est souvent de nature fibreuse et se forme généralement en cicatrice du tissu conjonctif (un processus appelé fibrose). Ainsi, la fibrose peut comprendre la fibrose pulmonaire, la fibrose rénale, la fibrose hépatique, la fibrose cutanée, la fibrose oculaire, la fibrose cardiaque, et d'autres états fibrotiques divers. Les hydrolysats de protéines selon l'invention peuvent également être utiles pour traiter d'autres états pathologiques à médiation par la furine, y compris, mais sans y être limités, l'hypertension, le cancer, des maladies infectieuses (bactériennes et virales) et des troubles génétiques (par exemple, la fibrose kystique (CF)), et des troubles neurodégénératifs (voir à ce propos le document WO2019215341 qui concerne de nouveaux composés inhibiteurs de furine convertase et les compositions pharmaceutiques les contenant, et qui cite de nombreuses publications démontrant l'activité pharmaceutique d'inhibiteurs, lesquelles citations sont incorporées par référence dans la présente description).

Par accord intergouvernemental du 10 avril 2018 entre le gouvernement de la République française et le Royaume d'Arabie Saoudite, la demanderesse, Agence Française Pour Le Développement d'AlUla (AFALULA) ainsi que la Commission Royale pour AIUIA (RCU) ont pour projet commun notamment de développer une agriculture responsable et l'économie locale, notamment par la production locale de produits naturels dérivés des plantes indigènes et de protéger la biodiversité et les droits de la région d'AlUla du Royaume d'Arabie Saoudite. Le Royaume d'Arabie Saoudite est membre du Protocole de Nagoya depuis le 8 octobre 2020. Au moment de la rédaction du présent brevet, le règlement d'exécution en vertu duquel le Protocole de Nagoya sera intégré dans les aspects pertinents du droit local est en cours d'examen. Par conséquent, à ce stade, le Royaume d'Arabie Saoudite n'a pas d'exigences spécifiques en ce qui concerne cette demande de brevet et le Protocole de Nagoya. Ainsi, au jour du dépôt de la demande de brevet, il n'y a aucune exigence de certificat de conformité concernant l'accès aux ressources génétiques.

### Résumé

L'invention a pour premier objet un procédé d'obtention d'un hydrolysat de protéines du tourteau des graines de *Moringa peregrina,* comprenant les étapes suivantes selon lesquelles :
a) on recueille les graines non décortiquées à maturité du fruit de *Moringa peregrina* que l'on sèche pour obtenir un taux d'humidité interne inférieur à 8%,
b) on presse les graines séchées de sorte à séparer l'huile du reste de la graine, de sorte à obtenir le tourteau comprenant moins de 6% en poids d'huile résiduelle,
c) on broie le tourteau obtenu à l'étape b),
d) on met en dispersion en phase aqueuse le tourteau broyé obtenu à l'étape c),
e) on réalise une protéolyse chimique de la dispersion aqueuse obtenue à l'étape d) pendant une durée d'environ 2 heures, à pH supérieur à 13 et à une température comprise entre 16 et 25°C,
f) on neutralise la protéolyse pour stabiliser l'hydrolysat de protéines obtenu,
g) on récupère l'hydrolysat de protéines par séparation solide/liquide,
h) on purifie l'hydrolysat de protéines par ultrafiltration et/ou nanofiltration, puis éventuellement,
i) on effectue une lyophilisation de l'hydrolysat de protéines obtenu à l'étape h).

L'invention a comme second objet un hydrolysat de protéines du tourteau des graines non décortiquées et récoltées à maturité du fruit de *Moringa peregrina* comprenant une fraction majoritaire de dérivés d'acides aminés, d'acides aminés, de peptides et glycopeptides dont le poids moléculaire P1 est compris entre 1 500 Da et 5 000 Da, une fraction d'environ 20% (masse/masse) dont le poids moléculaire P2 est compris entre 10 000 à 17 000 Da et une fraction d'environ 20% (masse/masse) dont le poids moléculaire P3 est d'environ 23 000 Da, en ce qu'il est obtenu par protéolyse chimique à pH supérieur à 13 pendant une durée d'environ 2 heures à une température comprise entre 16 et 25°C et en ce qu'il est liquide et présente une densité supérieure à 1 et préférentiellement autour de 1.1.

Grâce à ses caractéristiques, l'hydrolysat de protéines de *Moringa peregrina* selon l'invention n'a jamais été mis en évidence dans le genre *Moringa* et la famille Moringaceae. Il sera démontré que l'extrait de l'espèce *Moringa peregrina* présente un profil peptidique particulier et différent des autres espèces du genre, notamment de l'espèce *Moringa oleifera,* que la demanderesse a su mettre en évidence.

L'invention a pour troisième objet un hydrolysat de protéines du tourteau des graines de *Moringa peregrina* pour son application comme médicament.

L'invention a pour quatrième objet une composition pharmaceutique ou dermatologique comprenant à titre d'agent actif, une quantité efficace d'un hydrolysat de protéines du tourteau des graines de *Moringa peregrina* et un excipient physiologiquement acceptable.

L'invention a enfin pour cinquième objet une composition pharmaceutique ou dermatologique destinée à être utilisée pour le traitement de maladies fibrotiques, de la fibrose de la peau, le traitement de l'inflammation, du cancer, de maladies infectieuses de type virales et du lentigo.

### Brève description des figures

L'invention sera mieux comprise, et d'autres buts, détails, caractéristiques et avantages de celle-ci apparaîtront plus clairement au cours de la description suivante de plusieurs modes de réalisation particuliers de l'invention, donnés uniquement à titre illustratif et non limitatif, en référence aux dessins annexés.
[fig.1] représente un diagramme de l'infection d'un virus à couronne SRAS-COV2 (Syndrome Respiratoire Aigu Sévère-Coronavirus2) qui démontre l'effet anti-infectieux et virostatique de l'hydrolysat de protéines de *peregrina* selon l'invention contre les pseudovirions à couronne SRAS COV2.
[fig.2] représente le diagramme d'inhibition de la furine par un extrait de l'huile de peregrina. Dans cette figure, ** signifie significativement différent du groupe « contrôle » (P<0.001).
[fig.3] représente le diagramme d'inhibition de la furine par un extrait de tourteau de peregrina (éthanol 96°). Dans cette figure, * signifie significativement différent du groupe « contrôle » (P<0.001).
[fig.4] représente le diagramme d'inhibition de la furine par un hydrolysat de protéines de tourteau de peregrina selon l'invention. Dans cette figure, *** signifie significativement différent du groupe « contrôle » (P<0.001).

### Description des modes de réalisation

Dans cette description, à moins qu'il n'en soit spécifié autrement, il est entendu que, lorsqu'un intervalle est donné, il inclut les bornes supérieures et inférieures dudit intervalle.

Dans la présente invention, les abréviations suivantes signifient :
- MTT : bromure de 3-(4,5-diméthylthiazol-2-yl)-2,5-diphényl tetrazolium (le test MTT est une méthode rapide pour la numération des cellules vivantes)
- SDS : Sodium Dodecyl Sulfate
- PBS : Phosphate Buffer Saline
- ELISA : Enzyme-Linked Immunosorbent Assay
- PCR : Polymerase Chain Reaction
- ANOVA : Analysis Of Variance
- MSH : Melanocyte Stimulating Hormone

Dans la présente invention, on entend par :
- « majoritairement des dérivés d'acides aminés, des acides aminés, des peptides et glycopeptides » une quantité supérieure à 20%, préférentiellement encore supérieure à 30% et pouvant atteindre environ 40% (Masse/Masse) de matière sèche en dérivés d'acides aminés, acides aminés, peptides et glycopeptides, préférentiellement encore 50% du poids de la matière sèche.
- « quantité efficace » la quantité nécessaire de molécules actives pour obtenir le résultat recherché, à savoir, assurer l'activité thérapeutique recherchée.
- « protéolyse » la segmentation des protéines en peptides, oligopeptides et ses fragments de base (acides aminés) et ses résidus via l'hydrolyse chimique.
- « voie locale », le fait que le médicament, directement appliqué sur son lieu d'action, exerce son effet pharmacologique sur le site précis de l'affection. Le but de la voie locale est de limiter la diffusion du principe actif à partir de son lieu d'administration permettant un minimum d'effets indésirables. Les principales voies locales utilisées sont la voie cutanée, la voie nasale et respiratoire, la voie oculaire, la voie auriculaire, la voie vaginale et la voie buccale.
- « application topique », le fait d'appliquer ou d'étaler le principe actif selon l'invention, ou une composition le contenant, à la surface de la peau, d'une muqueuse ou des phanères.
- « physiologiquement acceptable » qui convient à une utilisation topique, en contact avec la peau humaine, ou à une utilisation par d'autres voies d'administration, par exemple la voie orale ou l'injection dans la peau, sans risque de toxicité, d'incompatibilité, d'instabilité, de réponse allergique.
- « tourteau », la partie déshuilée de la graine après pressage. Il s'agit du résidu solide de l'extraction de l'huile des graines. C'est un coproduit de la trituration, procédé de fabrication de l'huile. Il représente généralement de 50 à 75% de la masse des graines.
- « graines non décortiquées », le fait que la coque (péricarpe) et le tégument des graines récoltées soient maintenus autour des amandes.
- « à maturité du fruit », le fait que le fruit soit mûr, préférentiellement lorsque la gousse est en début de déhiscence et qu'elle prend une coloration beige foncé à brune et lorsqu'une torsion exercée de 180° sur le quart inférieur de la gousse provoque l'amorce de l'ouverture des valves.
- « environ » une marge plus ou moins de 10% à 20% par rapport à l'information donnée.
- « pool de molécules actives », « agent actif » et également « principe actif », l'hydrolysat de protéines extrait selon le procédé de l'invention à partir du tourteau des graines de *Moringa peregrina.* Cet hydrolysat est responsable des activités biologiques décrites dans la présente invention.
- « agent actif » une quantité suffisante d'un extrait selon l'invention pour obtenir les activités biologiques décrites. Selon si l'extrait est liquide ou séché, concentré ou non, les quantités de l'agent actif peuvent varier dans les proportions de 0,0001 à 40% en poids par rapport au poids total de la composition.

L'invention concerne en premier objet un procédé d'obtention d'un hydrolysat de protéines du tourteau des graines de *Moringa peregrina* comprenant les étapes suivantes selon lesquelles :
a) on recueille les graines non décortiquées à maturité du fruit de *Moringa peregrina* que l'on sèche pour obtenir un taux d'humidité interne inférieur à 8%,
b) on presse les graines séchées de sorte à séparer l'huile du reste de la graine de sorte à obtenir le tourteau comprenant moins de 6% en poids d'huile résiduelle,
c) on broie le tourteau obtenu à l'étape b),
d) on met en dispersion en phase aqueuse le tourteau broyé obtenu à l'étape c),
e) on réalise une protéolyse chimique de la dispersion aqueuse obtenue à l'étape d) pendant une durée d'environ 2 heures, à pH supérieur à 13 et à une température comprise entre 16 et 25°C,
f) on neutralise la protéolyse pour stabiliser l'hydrolysat de protéines obtenu,
g) on récupère l'hydrolysat de protéines par séparation solide/liquide,
h) on purifie l'hydrolysat de protéines par ultrafiltration et/ou nanofiltration effectuée avec un seuil de coupure compris entre 100 et 25000 Da.
   Puis, selon un mode de réalisation particulier,
i) on effectue une lyophilisation de l'hydrolysat de protéines obtenu à l'étape h).

On recueille les graines non décortiquées, c'est à dire dont on garde la coque à maturité du fruit et préférentiellement lorsque la gousse est en début de déhiscence.

On sèche les graines pour obtenir un taux d'humidité interne inférieur à 8% et préférentiellement autour de 6% ; le séchage se fera préférentiellement sur clayette ventilée à l'abri des rayons du soleil, de préférence sous ombrière à l'air libre.

On broie alors les graines séchées extemporanément au pressage à froid qui permet de séparer mécaniquement l'huile du reste de la graine compressée, à savoir le tourteau.

Le tourteau est alors broyé mécaniquement avec tout type de broyeur mécanique tel que système à marteaux, à fléau, à couteaux, à concassage/déchiquetage, à billes ou boulets ou à pilon, mais aussi avec tout type de cryobroyeur.

La dispersion en phase aqueuse selon l'étape d) et la protéolyse selon l'étape e) s'effectuent avantageusement toujours sous agitation permettant ainsi une dispersion et une homogénéisation du solide dans le liquide, améliorant ainsi la surface d'échange globale et par conséquent la protéolyse.

On obtient un hydrolysat de protéines liquides d'une densité supérieure à 1 et préférentiellement autour de 1.1, comprenant un taux de matières sèches compris entre 10 et 15 %, préférentiellement autour de 12.5%, comprenant entre 1 et 6% de composés azotés, notamment des dérivés nitriles volatiles dans une proportion de 0.5 à 1.5%, préférentiellement autour de 0,8%, ainsi que 20 mg/litre de polyphénols (0,002%).

Selon un mode de réalisation préférentiel du procédé selon l'invention, la température de la protéolyse de l'étape f) est d'environ 22°C.

Selon un mode de réalisation préférentiel du procédé selon l'invention, la séparation solide/liquide de l'étape g) est réalisée par différents procédés tels que centrifugation, essorage, filtration.

Dans un mode de réalisation du procédé d'obtention de l'hydrolysat de protéines de *Moringa peregrina* liquide obtenu, l'hydrolysat de protéines est purifié par distillation, microfiltration, ultrafiltration et/ou nanofiltration pour le concentrer en composés d'intérêt par rapport aux matières organiques également extraites, notamment aux autres dérivés également extraits. Ces étapes de purification permettent de concentrer le pool de composés d'intérêt au dépend d'autres composés extraits tels que cités.

Selon un autre mode de réalisation préférentiel du procédé selon l'invention, la nanofiltration est effectuée de manière à séparer 3 bandes ou fractions de l'hydrolysat de protéines comprenant une bande P1 dont le poids moléculaire est inférieur à 10 000 Da, une bande P2 dont le poids moléculaire est compris entre 10 000 et 17 000 Da et une bande P3 dont le poids moléculaire est d'environ 23 000 Da.

Il est avantageux également de séparer par nanofiltration les trois bandes afin d'obtenir par le procédé d'obtention d'un hydrolysat de protéines :
- une bande P1 avec une nanofiltration effectuée avec un seuil de coupure compris entre 1 500 Da et 5 000 Da, de préférence avec un seuil de coupure compris entre 3 000 Da et 4 500 Da.
- une bande P2 avec une nanofiltration effectuée avec un seuil de coupure compris entre 10 000 Da et 17 000 Da.
- une bande P3 avec une nanofiltration effectuée avec un seuil de coupure compris entre 17 000 Da et 25 000 Da.

Dans un autre mode de réalisation du procédé d'extraction selon l'invention, l'hydrolysat de protéines liquide obtenu est séché de manière à obtenir un hydrolysat sec du tourteau des graines de *Moringa peregrina* contenant une quantité supérieure à 10%, préférentiellement supérieure à 20%, préférentiellement encore supérieure à 30% et pouvant atteindre environ 40% (Masse/Masse) de matière sèche en peptides, oligopeptides, glycopeptides et en acides aminés ou leurs dérivés nitrilés volatiles, préférentiellement encore 50% du poids de la matière sèche.

Selon un mode de réalisation de l'invention, l'hydrolysat de protéines de tourteau des graines de *Moringa peregrina* liquide obtenu est séché par exemple par atomisation, lyophilisation ou zéodratation de manière à obtenir un hydrolysat de graines de *Moringa peregrina* solide, l'eau étant évaporée. Le séchage peut être réalisé en présence d'un support organique tel que la maltodextrine, cyclodextrine ou inuline, ou en présence d'un support inorganique tel que phyllosilicate, magnésium silicate ou carbonate et ses sels.

L'invention concerne également l'hydrolysat de protéines du tourteau des graines de *Moringa peregrina* obtenu par le procédé d'extraction selon l'invention.

L'invention a pour deuxième objet un hydrolysat de protéines du tourteau des graines non décortiquées et récoltées à maturité du fruit de *Moringa peregrina,* comprenant une fraction P1 majoritaire de dérivés d'acides aminés, d'acides aminés, de peptides et glycopeptides dont le poids moléculaire est compris entre 1 500 Da et 5 000 Da, une fraction P2 d'environ 20% (masse/masse) dont le poids moléculaire est compris entre 10 000 à 17 000 Da et une fraction P3 d'environ 20% (masse/masse) dont le poids moléculaire est d'environ 23 000 Da, en ce qu'il est obtenu par protéolyse chimique à pH supérieur à 13 pendant une durée d'environ 2 heures à une température comprise entre 16 et 25°C et en ce qu'il est liquide et présente une densité supérieure à 1 et préférentiellement autour de 1.1.

Selon un mode de réalisation, l'hydrolysat de protéines liquide obtenu est séché de manière à obtenir un hydrolysat sec du tourteau des graines de *Moringa peregrina* contenant une quantité supérieure à 20%, préférentiellement encore supérieure à 30% et pouvant atteindre environ 40% (Masse/Masse) de matière sèche en peptides, oligopeptides, glycopeptides et en acides aminés ou leurs dérivés nitrilés volatiles, préférentiellement encore 50% du poids de la matière sèche.

Selon un mode de réalisation préférentiel, l'hydrolysat de protéines comprend également entre 0.3 % et 3 % de composés volatiles, dont 50% de ces composés, soit entre 0.15% et 1.5% de l'extrait selon l'invention, est constitué de composés nitrilés légers, avec principalement l'isobutyronitrile et le méthyl-butanenitrile ; dont 5 à 10% de ces composés, soit entre 0.015 et 0.3% de l'extrait selon l'invention, est constitué de dérivés d'isothiocyanates, avec principalement l'isothiocyanate d'isopropyle et l'isothiocyanate d'isobutyle ; dont 1 à 5% de ces composés, soit entre 0.003 et 0.15 %, est constitué d'huile essentielle, avec principalement de l'Eucalyptol, du Menthol et du Benzaldéhyde.

Selon encore un autre mode de réalisation, l'hydrolysat de protéines comprend un taux de matières sèches compris entre 10 et 15 %, préférentiellement autour de 12.5% comprenant entre 1 et 6% de composés azotés, notamment des dérivés nitriles volatiles dans une proportion de 0.5 à 1.5%, préférentiellement autour de 0.8%, ainsi que 20 mg/litre de polyphénols.
Dans le cadre de la présente invention, la partie de plante choisie est la graine de *Moringa peregrina.* Il est connu que les graines de *Moringa peregrina* sont utilisées pour l'extraction de leur huile, utile pour l'autoconsommation ou en soins médicinaux traditionnels divers. Le tourteau obtenu après que la graine a été déshuilée, est un déchet actuellement utilisé pour la nourriture des animaux notamment.

Selon encore un autre mode de réalisation, l'hydrolysat de protéines comprend la fraction P1 majoritaire dont le poids moléculaire est compris entre 1 500 Da et 5 000 Da.

Selon encore un autre mode de réalisation, l'hydrolysat de protéines comprend la fraction P2 d'environ 20% (masse/masse) dont le poids moléculaire est compris entre 10 000 Da et 17 000 Da.

Selon encore un autre mode de réalisation, l'hydrolysat de protéines comprend la fraction P3 d'environ 20% (masse/masse) dont le poids moléculaire est d'environ 23 000 Da.

Selon encore un autre mode de réalisation, l'hydrolysat de protéines comprend la fraction P1 dont le poids moléculaire est compris entre 1 500 Da et 5 000 Da et la fraction P2 dont le poids moléculaire est compris entre 10 000 à 17 000 Da.

L'invention a pour troisième objet un hydrolysat de protéines du tourteau des graines de *Moringa peregrina* pour son application comme médicament.

L'invention a pour quatrième objet une composition pharmaceutique ou dermatologique comprenant à titre d'agent actif, une quantité efficace d'un hydrolysat de protéines du tourteau des graines de *Moringa peregrina* selon l'invention et un excipient physiologiquement acceptable.

Les compositions selon l'invention peuvent se présenter sous toutes les formes galéniques normalement utilisées selon que la composition doit être ingérée, injectée ou appliquée sur la peau ou les muqueuses.

Selon une première variante, les différentes compositions sont adaptées à l'ingestion ; la composition peut se présenter sous forme de capsules, de sirops, de granulés ou de comprimés. Elle pourra ne pas comprendre d'excipient et être constituée, en intégralité, de l'extrait végétal comprenant l'hydrolysat de protéines sous forme séchée.

Selon une deuxième variante, les différentes compositions sont adaptées à l'injection ; la composition peut se présenter sous forme d'une lotion aqueuse, huileuse ou sous forme de sérum.

Selon une troisième variante, les différentes compositions sont destinées plus particulièrement à une administration par voie locale, permettant un effet pharmacologique sur le site précis de l'affection via la peau où les muqueuses.

Les principales voies locales utilisées pour l'administration de l'agent actif selon l'invention sont la voie cutanée et percutanée, la voie nasale et respiratoire, la voie oculaire, la voie auriculaire et la voir vaginale. D'autres voies peuvent être envisagées, notamment la voie buccale, pour administrer les compositions via les muqueuses ainsi que la voie sous-cutanée par micro-injections.

Les compositions selon l'invention à visée pharmaceutique peuvent se présenter sous toutes les formes galéniques normalement utilisées pour une application par voie locale. Elles peuvent être administrées sur les muqueuses, notamment au niveau nasal ou respiratoire, oculaire, buccal, vaginal et auriculaire.

Les compositions selon l'invention à visée dermatologique peuvent se présenter sous toutes les formes galéniques normalement utilisées dans le domaine de la dermatologie pour une application par voie cutanée. Elles peuvent être administrées par voie transdermique ou appliquées topiquement sur la peau.

Les compositions incorporant un hydrolysat de protéines selon l'invention pourront comprendre des ingrédients couramment utilisés dans ce type de formulations par voie locale ou voie cutanée.

Selon un mode de réalisation préférentiel, les différentes compositions sont adaptées à l'administration topique et incluent les crèmes, les émulsions huile dans eau et eau dans huile, les laits, les pommades, les lotions, les huiles, les baumes, les solutions aqueuses ou hydro-alcooliques ou glycoliques, les sérums, les poudres, les patchs, les sprays ou tout autre produit pour application externe tel que par, exemple, les dispositifs médicaux ou les produits aérosol contenant également un agent propulseur sous pression.

Selon un autre mode de réalisation préférentiel, les différentes compositions sont adaptées à l'injection sous-cutanée et l'administration transdermique ; la composition peut se présenter sous forme d'une lotion aqueuse, émulsion ou sous forme de sérum. Dans les systèmes transdermiques ou patches, la libération du ou des principes actifs est contrôlée par une membrane perméable et généralement adhésive directement en contact avec la peau.

Les compositions selon l'invention destinées plus particulièrement à une administration par voie locale contiennent un milieu pharmaceutique ou dermatologique acceptable, c'est-à-dire compatible avec la peau et les muqueuses, et couvrent toutes les formes galéniques appropriées. Ces compositions pourront notamment être sous forme de crèmes, émulsions huile-dans-eau, ou eau-dans-huile ou émulsions multiples, sérums, solutions, suspensions, gels, laits, lotions, sticks, aérosols, sprays ou tout autre produit pour application externe tel que, par exemple, les dispositifs médicaux ou les produits aérosol contenant également un agent propulseur sous pression, ou encore sous forme de poudres, et adaptées à une application sur la peau et les muqueuses. Ces compositions comprennent les excipients nécessaires à leur formulation, tels que solvants, émollients, épaississants, diluants, tensioactifs, anti-oxydants, agents bioactifs, colorants, conservateurs, parfums.

Les compositions selon l'invention comprennent donc tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs dermatologiques ou pharmaceutiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées des compositions selon l'invention.

Dans les compositions de l'invention, l'hydrolysat de protéines selon l'invention est utilisé en une quantité allant de 0.0001 à 40% en poids par rapport au poids total de la composition.

Dans un mode de réalisation préférentiel, l'hydrolysat de protéines selon l'invention est utilisé en une quantité allant de 0.001 à 10% en poids par rapport au poids total de la composition, de préférence encore en une quantité allant de 0,01% à 5% en poids par rapport au poids total de la composition.

Enfin, l'invention a pour cinquième objet une composition pharmaceutique ou dermatologique pour son utilisation en tant que médicament pour le traitement :
- de maladies fibrotiques et le traitement de l'inflammation, comprenant comme agent actif une quantité efficace de la fraction P1 ou P2 de l'hydrolysat de protéines, de préférence la fraction P1.
- du cancer, comprenant comme agent actif une quantité efficace de la fraction P3 de l'hydrolysat de protéines.
- de maladies infectieuses de type virales, et notamment pour inhiber les protéines Spike-COV2 du SRAS-COV2, comprenant comme agent actif une quantité efficace de l'hydrolysat de protéines dans son ensemble.
- du lentigo, comprenant comme agent actif une quantité efficace des fractions P1 et P2 de l'hydrolysat de protéines.

Par « dérive génétique » on entend notamment le stress environnemental.

Pour le traitement de maladies fibrotiques, on entend par fibrose la fibrose pulmonaire, la fibrose rénale, la fibrose hépatique, la fibrose cutanée, la fibrose oculaire, la fibrose cardiaque, et d'autres états fibrotiques divers et pour le traitement d'autres états pathologiques à médiation par la furine, notamment, mais sans y être limités, l'hypertension, le cancer, les maladies infectieuses y compris virales et bactériennes, des troubles génétiques (par exemple, la fibrose kystique (CF)), et des troubles neurodégénératifs.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention.

L'usage du verbe « comporter », « comprendre » ou « inclure » et de ses formes conjuguées n'exclut pas la présence d'autres éléments ou d'autres étapes que ceux énoncés dans une revendication.

### Exemples

### Exemple 1 : Préparation d'un hydrolysat de protéines végétal selon l'invention à partir du tourteau de Moringa peregrina

Des graines de *Moringa peregrina* (Forssk.) Fiori ont été séchées pour obtenir un taux d'humidité interne inférieur à 8% et préférentiellement autour de 6%, puis pressées avec une presse mécanique à vis sans fin, de sorte à séparer l'huile du reste de la graine pour obtenir d'une part l'huile vierge et d'autre part un tourteau. Le tourteau est alors isolé sous forme de boudins prédécoupés en morceau de 1 à 2 cm sur lequel est effectué l'extraction.

Les matières premières utilisées sont les suivantes.

**[Tableau 1]**

| **Matières** | **%** | **Qté réelle pesée** |
|---|---|---|
| Tourteau de *peregrina* prédécoupé en morceaux de 1 à 2 cm | 8.4% | 33.6 |
| Eau du réseau | 80.68% | 322.7 |
| Hydroxyde de sodium | 3,36 % | 13.5 |
| Acide citrique monohydrate F6000 | 7.14% | 28.7 |
| Benzoate de sodium | 0.42% | 1.7 |
| | 100 | 400.2 |

### Protocole :

a) On prépare une solution concentrée à 1 molaire d'un agent alcalin fort tel que notamment l'hydroxyde de sodium, l'hydroxyde de potassium ou l'hydroxyde de calcium, ou d'un mélange aqueux concentrant 1 molaire d'agents alcalins forts, mais préférentiellement l'hydroxyde de sodium ; cette solution alcaline forte présente un pH entre 13 et 14,
b) On pèse 9.1% (masse/masse) de tourteau de *peregrina* prédécoupé en morceaux de 1cm environ dans 90.9% (masse/masse) de la solution alcaline,
c) on broie le tourteau obtenu à l'étape b),
d) On met en dispersion en phase aqueuse le tourteau broyé obtenu à l'étape c),
e) on réalise une protéolyse chimique de la dispersion aqueuse obtenue à l'étape d) pendant une durée de 2 heures et à une température de 22°C,
f) on neutralise la protéolyse pour stabiliser l'hydrolysat de protéines obtenu,
g) on récupère l'hydrolysat de protéines par séparation solide/liquide par passage sur filtre 1 µm,
h) on purifie l'hydrolysat de protéines par ultrafiltration,

On obtient un filtrat jaune translucide contenant environ 12,48% de matière sèche. L'extrait liquide obtenu est nommé par la suite « hydrolysat de protéines *peregrina* selon l'invention » ou « hydrolysat de protéines *peregrina »* ou « extrait selon l'invention ».

Cet hydrolysat de protéines *peregrina* selon l'invention présente une densité supérieure à 1 et préférentiellement autour de 1.1, comprenant un taux de matières sèches compris entre 10 et 15 %, préférentiellement autour de 12.5%, comprenant entre 1 et 6% de composés azotés, notamment des dérivés nitriles volatiles dans une proportion de 0.5 à 1.5%, préférentiellement autour de 0.8% et 20 mg/litre de polyphénols. La composition de l'hydrolysat de protéines *peregrina* sec selon l'invention est donnée ci-dessous.

**[Tableau 2]**

| Tr | N° CAS | Composés | % MS |
|---|---|---|---|
| 4.42. | 64-17-5 | Ethanol | 0.809 |
| 5.58 | 67-64-1 | Acétone | 0.444 |
| 6.10 | 75-15-0 | Disulfure de carbone | 0.327 |
| 7.83 | 78-93-3 | 2-Butanone | 0.165 |
| 8.57 | 141-78-6 | Acétate d'éthyle | 0.047 |
| 8.99 | 78-82-0 | Isobutyronitrile | 20.720 |
| 10.39 | 78-82-0 | Isobutyronitrile | 0.127 |
| 12.06 | 547-63-7 | Isobutyrate de méthyle | 0.201 |
| 14.10 | 18936-17-9 | 2-Méthyl-butanenitrile | 2.194 |
| 14.56 | 625-28-5 | 3-Méthyl-butanenitrile | 27.495 |
| 18.77 | 66-25-1 | Hexanal | 0.186 |
| 21.09 | 2253-73-8 | Isothiocyanate d'isopropyle | 4.590 |
| 23.65 | 628-73-9 | Hexanenitrile | 0.198 |
| 24.43 | 110-43-0 | 2-Heptanone | 0.093 |
| 27.18 | 4426-79-3 | 2-isothiocyanato-Butane | 1.058 |
| 27.59 | 80-56-8 | Alpha-Pinène | 0.048 |
| 28.45 | 591-82-2 | Isothiocyanate d'isobutyle | 2.299 |
| 28.99 | 100-52-7 | Benzaldéhyde | 1.753 |
| 29.77 | 108-95-2 | Phénol | 0.413 |
| 30.80 | 13475-82-6 | 2,2,4,6,6-pentamethyl-Heptane | 0.318 |
| 32.80 | 99-87-6 | Para-Cymène | 0.232 |
| 33.10 | 138-86-3 | Limonène | 0.133 |
| 33.34 | 470-82-6 | Eucalyptol | 0.918 |
| 36.45 | 1195-32-0 | Para-Cyménène | 0.035 |
| 36.88 | 124-19-6 | Non anal. | 0.080 |
| 40.26 | 65-85-0 | Acide benzoique | 19.150 |
| 41.07 | 1490-04-6 | Menthol | 0.918 |
| 56.29 | 96-76-4 | 2,4-Di-telt-butylphenol | 0.086 |
| | | Total | 85.035 |

L'hydrolysat de protéines *peregrina* contient de l'isothiocyanate d'isopropyle et de l'isothiocyanate d'isobutyle à des niveaux relativement élevés, confirmant les publications antérieures sur l'espèce (KJAER, A. et al. 1979, Isothiocyanates in Myrosinase-treated seed extracts of Moringa peregrina, Phytochemistry, 18, p. 1485-1487 ; AFSHARYPUOR, S. et al., 2010, Volatile Constituents of the Seed Kernel and Leaf of Moringa peregrina (Forssk.) Fiori, Agricolt. Cultivated in Chabahar (Iran), Iranian Journal of Pharmaceutical Sciences 6(2): p. 141-144 ; DEHSHAHRI, S. et al., 2012, Determination of volatile glucosinate degradation products in seed coat, stem and in vitro cultures of Moringa peregrina (Forssk.) Fiori, ScienceOpen, Research in Pharmaceutical Sciences 7(1) : p. 51-56). Les isothiocyanates sont des composés produits par diverses plantes appartenant à l'ordre des Brassicales, notamment dans les familles des Brassicaceae, Capparaceae, Caricaceae et Moringaceae comme système de défense contre les attaques de pathogènes. Dans le genre *Moringa,* ils ont été identifiés notamment dans *M. oleifera* Lam. et *M. stenopetala* (Baker f.) Cufold. (ABD RANI, N.Z. et al., 2018, Moringa genus: A review of Phytochemistry and Pharmacology, Frontiers in Pharmacology, vol. 9, art. 108, p. 3-8). Les isothiocyanates proviennent de l'hydrolyse des glucosinolates par l'enzyme myrosinase lorsque les tissus de la plante sont endommagés. Il a été rapporté que les isothiocyanates ont divers effets biologiques, tels qu'une activité antifongique (TRONCOSO-ROJAS, R. et al., 2007, Natural compounds to control fungal diseases in fruits & vegetables, in TRONCOSO-ROJAS, R., TIZNADO-HERNANDEZ, M. E., GONZALEZ-LEON, A. (éd) Recent advances in alternative postharvest technologies to control fungal diseases in fruits & vegetables. Transworld Research Network, Kerala, India, p. 127-156 ; TRONCOSO-ROJAS, R. et al., 2005, Analysis of the isothiocyanates present in cabbage leaves extract and their potential application to control Alternaria rot in bell peppers, Food Research International 38, p. 701-708), des effets anti-microbiens, anti-cancéreux et anti-inflammatoires (PARK, E. J. et al., 2011, Inhibition of lipopolysaccharide induced cyclooxygenase-2 expression and inducible nitric oxyde synthase by 4-[(2'-O-acetyl-α-L-rhamnosyloxy)benzyl]isothiocyanate from M. oleifera, Nutrition and Cancer 63(6), p. 971-982 ; RAJAN T. S. et al., 2016, Anticancer activity of glucomoringin isothiocyanate in human malignant astrocytoma cells, Fitoterapa 110, p. 1-7 ; PADLA, E. P. et al. 2012, Antimicrobial isothiocyanates from the seeds of Moringa oleifera Lam., Zeitschrift für Naturforschung C, 67, p. 557-564 ; WATERMAN, C. et al., 2014, Stable, water extractable isothiocyanates from Moringa oleifera leaves attenuate inflammation in vitro. Phytochemistry 103, p. 114-122). Le furfural est présent à une concentration très standard, trouvée dans ce type de graines et dans de nombreux fruits secs. Le disulfure de carbone, l'isobutyronitrile, l'isobutyrate de méthyle, le méthyl butanenitrile et l'hexanenitrile sont des composés volatils issus des acides aminés. Ce sont des marqueurs de la dégradation des protéines. Ces composés de dégradation représentent environ 50% des composés volatils de l'hydrolysat de protéines de *peregrina.* Ce résultat indique que l'hydrolysat est principalement constitué de protéines. Il n'a été détecté que de très faibles traces de graisses ou de sucres libres dans l'hydrolysat de protéines *peregrina* ; l'hydrolysat est principalement constitué de protéines et de glycoprotéines. Dans la matière sèche, le tampon citrate (qui est un composé osidique) représente environ 50% du reste et englobe les composés glycosylés (osidiques) résultant de la dégradation par protéolyse des protéines, oligopeptides et acides aminés. Enfin, il est à noter la présence d'environ 21 mg/litre de polyphénols (0,002%) issus des graines de *Moringa peregrina.* L'acide benzoïque n'est pas à considérer dans la caractérisation car il s'agit d'un agent stabilisant ajouté à l'extrait.

L'extrait sec décrit ci-dessus est obtenu par une méthode gravimétrique basée sur la masse avant et après évaporation présent dans l'extrait liquide.

### Exemple 2 : Effet de l'hydrolysat de protéines peregrina selon l'invention comme inhibiteur de la furine convertase (dite furine)

L'objectif de cette étude est d'évaluer l'activité inhibitrice de l'hydrolysat de protéines du tourteau de la graine de *Moringa peregrina,* obtenu selon l'exemple 1.

Protocole : L'étude est réalisée en utilisant une furine humaine recombinante, qui catalyse le clivage d'un substrat spécifique fluorescent de référence.

Le décanoyl-Arg-Val-Lys-Arg-CMK à 100 nm a été utilisé comme inhibiteur de référence de l'activité de la furine.

La furine a été pré-incubée pendant 10 minutes à température ambiante en l'absence (contrôle) ou en présence du produit de référence ou de concentrations croissantes du composé testé :
« Hydrolysat de protéines *peregrina »* ; 0.02; 0.2 et 2% (v / v).

À la fin de l'étape de pré-incubation, un substrat de furine a été ajouté et les conditions expérimentales ont été incubées à nouveau à température ambiante à l'abri de la lumière pendant 5 minutes. Toutes les expériences ont été réalisées en triple.

### Préparation des composés:

L'hydrolysat de protéines *peregrina* testé a été solubilisé directement dans le tampon de dosage puis dilué afin d'atteindre la concentration à tester décrite ci-dessus.

### Protocole d'évaluation

Le clivage du substrat fluorescent de furine a été suivi pendant 5 minutes après l'addition du substrat en lisant la fluorescence à 485 nm / 535 nm.

### Statistiques

Les résultats sont exprimés en RFU (unités fluorescentes relatives) +/- S.D. (Écart-type).

La signification statistique de la différence observée entre les groupes « Contrôle » et « Produit de référence » a été évaluée par un test t de Student (p <0,001).

La signification statistique de la différence observée entre les groupes « Contrôle » ou « Témoin » et « Composé d'essai » a été évaluée par une analyse ANOVA unidirectionnelle, suivie d'un test HolmSidak (p <0,05).

L'inhibiteur de référence de la furine nommé Decanoyl-Arg-Val-Lys-Arg-CMK, testé à 100 nm, a significativement inhibé l'activité de la furine de 97,9% (p <0,001).

Ce résultat était attendu et valide l'étude.

Les résultats de l'activité de l'enzyme furine par rapport à l'activité basale obtenus sont donnés ci-après.

**[Tableau 3]**

| | |
|---|---|
| « Hydrolysat de protéines *peregrina »* à 0.02% (v/v) | 124.9% (p<0.001) |
| « Hydrolysat de protéines *peregrina »* à 0.2% (v/v) | 73.2% (p<0.001) |
| « Hydrolysat de protéines *peregrina »* à 2.0% (v/v) | 1.2% (p<0.001) |

Conclusion : A une concentration de 2%, l'hydrolysat de protéines *peregrina* selon l'invention est capable d'inhiber à 98,8% l'activité de la furine convertase. Dès la concentration de 0.2%, l'hydrolysat de protéines *peregrina* selon l'invention est capable d'inhiber l'activité de la furine de 26.8%.

### Exemple 3 : Effet de l'hydrolysat de protéines peregrina selon l'invention pour inhiber les enzymes Histones Desacétylases (HDACs) et Sirtuine I

L'objectif de cette étude est de démontrer l'activité inhibitrice de l'hydrolysat de protéines *peregrina* selon l'invention sur les enzymes HDACs et Sirtuines I, enzymes impliquées dans le contrôle de la dérive génétique par la régulation de la condensation/ décondensation de la chromatine qui donne accès ou non aux gènes portés par l'ADN. Une solution tamponnée de HDACs & Sirtuine I réagit avec un substrat pendant 20 minutes à 37°C et le transforme pour former un composé qui se colore en présence d'un développeur après incubation à 37°C pendant 10 minutes. L'activité maximum de désacétylation des Sirtuines peut ainsi être évaluée par mesure de l'absorbance à 405 nM. L'hydrolysat de protéines *peregrina* selon l'invention ou le produit de référence « Trichostatin A (STA) inhibiteur 1µM » sont mis en contact avec la solution de Sirtuines en même temps que le substrat de l'enzyme pendant 20 minutes à 37°C ; le substrat transformé par l'enzyme est coloré par l'ajout d'un développeur. L'activité désacétylante des HDACs et Sirtuine I en présence de l'actif est alors évaluée par mesure de l'absorbance à 405 nm. La modulation de cette activité est exprimée en pourcentage d'inhibition ou d'activation de l'activité maximum des HDACs & Sirtuine I en absence d'actif, c'est-à-dire uniquement en présence du substrat des enzymes HDACs & Sirtuine I.

Protocole : Une solution d'enzymes Sirtuines est incubée dans son substrat pendant 20 mn en absence (contrôle) ou présence du produit de référence, ou de concentrations croissantes des produits à l'essai. L'hydrolysat de protéines *peregrina* selon l'invention est testé aux concentrations suivantes : 2% ; 1% ; 0.1% (V/V). A la fin de la période d'incubation, l'activité des enzymes Sirtuines avec et sans produit à l'essai ou de référence a été révélée par coloration à l'aide d'une solution de développeur (10 mn à 37°C) et évaluée par mesure de l'absorbance des milieux réactionnels à 405 nm. Pour chaque concentration testée, la modulation de l'activité désacétylante des enzymes Histones Désacétysases et Sirtuines I par le produit à l'essai est calculée selon la formule suivante. Pourcentage de modulation de l'activité des enzymes Sirtuines = 100 x [(DO405 produit à l'essai ou de référence) - (DO405 HDACs & Sirtuine I seules)]/DO405 Sirtuines seules.

Si le résultat est négatif, le pourcentage est exprimé en inhibition de la réaction enzymatique ; si le résultat est positif, le pourcentage est exprimé en activation de la réaction enzymatique. Les résultats de l'inhibition des enzymes Histones Desacétylases (HDACs) sont donnés ci-après.

**[Tableau 4]**

| | | |
|---|---|---|
| Hydrolysat de protéines *peregrina* selon l'invention | Pourcentage | Inhibition versus Contrôle (%) |
| | 2% | 15 |
| | 1% | ns |
| | 0.10% | -18 |

Conclusion : A 2%, l'hydrolysat de protéines *peregrina* selon l'invention montre une inhibition significative des HDACs ; cette inhibition révèle la capacité de promouvoir l'autoprotection des cellules de la peau contre la dérive génétique. Ainsi l'extrait apparaît être utile contre l'une des dérives génétiques les plus courantes à la surface de la peau à savoir la fibrose qui se manifeste par l'apparition de « bouton » de chair (protubérance fibrotique). L'extrait pourra avantageusement interférer contre les phénomènes de fibrose à la surface de la peau.

### Exemple 4 : Effet de l'hydrolysat de protéines peregrina selon l'invention pour inhiber l'action de l'endothéline 1 (ET-1)

L'endothéline est une hormone peptidique dérivée des cellules endothéliales capable d'agir sur divers cellules et tissus via ses récepteurs. Par exemple, l'endothéline est connue pour provoquer une augmentation de la concentration intracellulaire de calcium dans les cellules musculaires vasculaires lisses et d'autres cellules (OHBA T. et al., WO2012/081370).

Il a été rapporté ces dernières années que l'endothéline de type 1 (ET-1) est un facteur bioactif qui contracte en permanence les muscles vasculaires et non vasculaires lisses par des actions directes et indirectes. On pense qu'une augmentation de l'action de l'endothéline fournit une vasoconstriction continue aux vaisseaux sanguins dans les sites périphériques, les reins et le cerveau, et serait à l'origine de diverses maladies telles que l'hypertension, l'infarctus du myocarde, les accidents vasculaires cérébraux, l'insuffisance rénale aiguë, le syndrome de Raynaud, l'athérosclérose, l'asthme et le cancer de la prostate (MIYAGAWA K. & EMOTO N. et al., 2014, Current state of endothelin receptor antagonism in hypertension and pulmonary hypertension, Therapeutic Advances in Cardiovascular Diseases, vol. 8(5) 202-216 ; SCHINZARI F. et al., 2018, Increased Endothelin-1-Mediated Vasoconstrictor Tone in Human Obesity : Effects of Gut Hormones, Physiological Research 67 suppl.1 : S69-S81). Trois types de peptides de la famille de l'endothéline avec des structures similaires, sont présents chez les animaux, y compris les humains (KADONO, S. et al., 2001, The Role of the Epidermal Endothelin Cascade in the Hyperpigmentation Mechanism of Lentigo Senilis, Journal of Investigative Dermatology 116 4, p. 571-577 ; ANONYMOUS, 2006, American Society for Biochemistry and Molecular Biology, New Cosmetics Handbook, p. 527-529). Tous ces peptides ont un effet vasoconstricteur et des actions vasopressives.

Ces dernières années, le rôle de l'endothéline dans diverses cellules autres que les cellules musculaires lisses vasculaires a été élucidé. Les publications scientifiques, par exemple, ont rapporté que la génération de ET-1 et d'autres facteurs augmentent dans les kératinocytes lorsque la peau est exposée à une irradiation UV, et ont suggéré que ET-1 soit associé à la mélanogenèse dans les mélanocytes exposés à une irradiation UV. En conséquence, la suppression de l'expression de l'endothéline est considérée comme utile, non seulement pour la prévention et/ou le traitement des maladies susmentionnées, mais également pour la prévention ou l'amélioration de la pigmentation de la peau (IMOKAWA, G. et al., 1995, Endothelin-1 as a New Melanogen: Coordinated Expression of its Gene and the Tyrosinase Gene in UVB-Exposed Human Epidermis, Journal of Investigative Dermatology 1051, p. 32-37 ; IMOKAWA, G. et al., 1992, Endothelins Secreted from Keratinocytes are Intrinsic Mitogens for Human Melanocytes, Journal of Biological Chemistry, 267, p. 24675-24680 ; GILCHREST, B. et al., 1996, Mechanisms of Ultraviolet Light-Induced Pigmentation, Photochemistry and Photobioliogy63, p. 1-10).

L'objectif est de doser l'endothéline de type 1 dans les cellules endothéliales micro-vasculaires humaines après exposition pendant 24h à l'hydrolysat de protéines *peregrina* selon l'invention.

Protocole : Les cellules endothéliales micro-vasculaires humaines ont été fournies par la société PELOBiotech et mises en culture dans les plaques 96 puits selon les procédures de production du fournisseur. Il s'agit de laisser agir les extraits à différentes concentrations sur les cellules endothéliales à 80% de confluence pendant 24 heures, puis de quantifier l'endothéline 1 à l'aide du kit ELISA PicoKine (EDN1) dans les surnageants cellulaires. Un test de viabilité au préalable est réalisé pour définir les doses non-toxiques à utiliser lors de dosage de l'endothéline 1. Le contrôle négatif est réalisé à l'aide des cellules en milieu de culture sans traitement. Le contrôle positif dans le test de viabilité est le SDS à 0,5%. Toutes les conditions sont préparées dans des milieux de culture, et les cellules sont ensuite incubées à 36.5°C / 5% CO2 pendant 24 heures.

### a) Application des solutions à tester sur les cellules endothéliales :

Les produits à tester sont mis en contact avec les cellules endothéliales en subconfluence dans les plaques 96 puits. Pour chaque concentration le test est réalisé sur 3 puits. Les plaques sont incubées pendant 24 heures ± 1 heure à 36,5°C / 5% CO2.

### b) Test de viabilité :

La viabilité cellulaire est évaluée par la méthode MTT sur les cellules après incubation avec les produits. Après 24 heures d'incubation, les surnageants sont récupérés et conservés à -20°C pour les dosages. Les puits sont ensuite rincés 1 fois avec 200 µL de PBS. Dans chaque puits 50 µL de solution de MTT à 0,5 mg/ml sont ajoutés : incubation pendant 3 heures à 36,5°C / 5% CO2. Dans chaque puits, 100 µL d'isopropanol sont ajoutés. Après homogénéisation, une lecture de l'absorbance est réalisée à 550 nm. Pour chaque condition, le rapport de la moyenne des densités optiques des cellules sur la moyenne des densités optiques des contrôles négatifs déterminera le taux de viabilité.

### c) Dosage de l'endothéline 1 :

Le dosage est réalisé à l'aide du kit ELISA. Les résultats de l'inhibition de l'action de l'endothéline sont donnés ci-après.

**[Tableau 5]**

| | Extrait concentration | Croissance cellulaire versus contrôle (%) | Endothéline 1 versus contrôle (%) | Endothéline 1 versus contrôle (*pg*/*ml*) |
|---|---|---|---|---|
| Hydrolysat de protéines selon l'invention | 2% | +8,46 | -34,90 | -47,09 |
| | 1% | 1,54 | -13,03 | -17,58 |
| | 0,10% | -1,15 | -2,44 | -16,79 |

Conclusion : Le test de viabilité réalisé à la fin du traitement n'a pas montré d'effet toxique pour les concentrations testées.

Le dosage de l'endothéline 1 est réalisé dans les surnageants cellulaires à des concentrations non toxiques. La quantité de l'endothéline 1 pour chaque condition est dosée à l'aide du kit ELISA.

Pour les cellules du contrôle du taux basal, les valeurs sont de l'ordre de 134,94 pg/ml. Pour les cellules traitées avec différentes concentrations d'hydrolysat, le taux basal se voit minoré de 47,09 pg/ml (avec 2% de l'extrait selon l'invention) et se voit minoré de 17,58 pg/ml (avec 1% de l'extrait selon l'invention). Ceci démontre des inhibitions très significatives dès 1% de l'extrait selon l'invention avec environ 13% d'inhibition de production d'endothéline de type 1 et jusque 34,90% d'inhibition avec 2% de l'extrait selon l'invention.

Les résultats sur l'endothéline et son inhibition dose-dépendante spécifique démontrent que l'hydrolysat de protéines selon l'invention présente un effet antiangiogenèse et anti-fibrotique, en raison de sa capacité à diminuer significativement l'endothéline.

Une étude complémentaire pour l'évaluation de P1, P2, P3 et du complexe P1+P2 décrits à l'exemple 12 est décrite ci-après sur la production d'endothélines : Modèle de test cellulaire sur Cellules endothéliales humaines normales.

Comme décrit dans l'exemple 12, il a été isolé de l'hydrolysat selon l'invention 3 bandes ou fractions protéiques caractérisées par leur masse P1 (a un poids moléculaire inférieur à 10 000 Da), P2 (a un poids moléculaire compris entre 10 000 et 17 000 Da) et enfin P3 (a un poids moléculaire d'environ 23 000 Da). Ces fractions sont dénommées ci-après également « extraits ».

**[Tableau 6]**

| | | **Témoin** | **Extrait P1 (%, v/v)** | | |
|---|---|---|---|---|---|
| | | | **0.1** | **0.3** | **1** |
| Endothéline-I (pg/µg de protéines) | | 33.8 | 29.0 | 44.0 | 38.3 |
| | | 36.7 | 34.2 | 41.5 | 31.7 |
| | | 39.7 | 30.1 | 38.1 | 33.9 |
| | **Moyenne** | **36.7** | **31.1*** | **41.2*** | **34.6*** |
| | Ecart Type | 3.0 | 2.7 | 2.9 | 3.3 |
| | **% du témoin** | **100.0** | **84.6** | **112.1** | **94.2** |

| | | | | | |
|---|---|---|---|---|---|
| *Significativité statistique (p>0.05) | | | | | |

A toutes les concentrations, les écarts types sont élevés, et la fraction P1 de l'hydrolysat de protéines ne présente pas de modulation significative sur l'endothéline de type I.

**[Tableau 7]**

| | | **Témoin** | **Extrait P2 (% v/v)** | | |
|---|---|---|---|---|---|
| | | | **0.1** | **0.3** | **1** |
| Endothéline-I (pg/µg de protéines) | | 33.8 | 34.8 | 24.7 | 34.6 |
| | | 36.7 | 35.7 | 28.9 | 39.3 |
| | | 39.7 | 34.0 | 29.2 | 40.8 |
| | **Moyenne** | **36.7** | **34.9*** | **27.6**** | **38.3*** |
| | Ecart Type | 3.0 | 0.9 | 2.5 | 3.2 |
| | **% du Témoin** | **100.0** | **94.9** | **75.2** | **104.1** |

| | | | | | |
|---|---|---|---|---|---|
| *Significativité statistique (p>0.05) **Significativité statistique (p<0.05) | | | | | |

Aux concentrations 0,1% et 1%, les écarts types sont élevés, et la fraction P2 de l'hydrolysat de protéines ne présente pas de modulation significative sur l'endothéline de type I à ces concentrations. A la concentration de 0,3%, P2 inhibe de 24,8% la production d'endothéline de type I.

**[Tableau 8]**

| | | **Témoin** | **Extraits P1+P2 (50/50) (%, v/v)** | | |
|---|---|---|---|---|---|
| | | | **0.1** | **0.3** | **1** |
| Endothéline-I (pg/µg de protéines) | | 33.8 | 39.4 | 42.0 | 34.5 |
| | | 36.7 | 29.7 | 34.2 | 34.7 |
| | | 39.7 | 42.2 | 38.3 | 37.9 |
| | **Moyenne** | **36.7** | **37.1*** | **38.1*** | **35.7*** |
| | Ecart Type | 3.0 | 6.6 | 3.9 | 1.9 |
| | **% du Témoin** | **100.0** | **100.9** | **103.8** | **97.2** |

| | | | | | |
|---|---|---|---|---|---|
| *Significativité statistique (p>0.05) | | | | | |

A toutes les concentrations, les écarts types sont élevés, et la combinaison des fractions P1 + P2 ne présente pas de modulation significative sur l'endothéline de type I.

**[Tableau 9]**

| | **Témoin** | **Extrait P3 (%, v/v)** | | |
|---|---|---|---|---|
| | | **0.1** | **0.3** | **1** |
| Endothéline-I (pg/µg of proteins) | 33.8 | 36.2 | 19.5 | 18.8 |
| | 36.7 | 38.9 | 14.8 | 20.4 |
| | 39.7 | 31.5 | 18.2 | 17.6 |
| **Moyenne** | **36.7** | **35.5*** | **17.5***** | **18.9***** |
| Ecart Type | 3.0 | 3.8 | 2.4 | 1.4 |
| **% du Témoin** | **100.0** | **96.7** | **47.7** | **51.5** |

| | | | | |
|---|---|---|---|---|
| *Significativité statistique (p>0.05) ***: Significativité statistique (p<0.001) | | | | |

Aux concentrations de 0,3% et de 1%, la fraction P3 de l'hydrolysat de protéines inhibe de manière très significative d'environ 50% la production d'endothéline de type I.

Conclusion : Les composés dénommés « Extrait P1 » et « Extrait P2 » n'ont pas d'action significative sur la modulation de l'endothéline de type 1 et seul l'« Extrait P3 » diminue significativement l'endothéline 1 libérée dans le milieu de culture par les cellules endothéliales normales humaines en culture monocouche avec un score de 52,3% d'inhibition avec 0.3% de l'extrait selon l'invention. Aucune synergie n'est constatée lorsque P1 et P2 sont combinées.

L'extrait P3 démontre spécifiquement une activité anti-cancéreuse (BAGNATO A. et al., 2011, Role of the endothelin axis and its antagonists in the treatment of cancer, British Journal of Pharmacology, 163: 220-233).

### Exemple 5 : Effet de l'hydrolysat de protéines peregrina selon l'invention pour protéger les cellules souches

Les tissus adultes, y compris l'épiderme cutané, l'épithélium gastro-intestinal et le système hématopoïétique, ont un taux élevé de renouvellement cellulaire. Le processus physiologique de maintien de l'homéostasie tissulaire est attribué au maintien d'un nombre constant de cellules dans le renouvellement des organes. Les ESC (cellules souches embryonnaires) sont essentielles pour le maintien et la régénération des tissus cutanés.

L'épiderme se développe à partir de l'ectoderme de surface embryonnaire. Il commence comme une seule couche de cellules progénitrices non spécifiées couvrant l'embryon après la neurulation et se développe dans la couche basale épidermique. La couche basale épidermique est enrichie en CSE (Cellules Souches Epidermales). En effet, les cellules de cette couche donnent naissance à toutes les structures épidermiques, y compris l'épiderme stratifié (également appelé épiderme interfolliculaire) et les appendices épidermiques, tels que les follicules pileux, les glandes sébacées et les glandes sudoripares. Le derme sous-jacent dérive principalement du mésoderme sous l'ectoderme. Le mésoderme est la principale source de cellules souches mésenchymateuses qui donnent naissance à des fibroblastes producteurs de collagène, des adipocytes sous-cutanés et des cellules immunitaires de la peau.

Les cellules souches sont des cellules indifférenciées dites pluripotentes avec un jeune génotype capable de s'auto-renouveler et de se différencier pour produire un organe ou un tissu, comme la peau. À ce stade, elles sont identifiées comme des « cellules multipotentes ». Étant donné que 50% de la descendance de la population de cellules souches reste indifférenciée, les cellules souches contribuent à préserver l'homéostasie et à assurer le renouvellement des cellules différenciées endommagées ou sénescentes. Or ces cellules souches épidermiques sont fréquemment affectées par l'environnement. Le stress oxydatif, comme la pollution ou le rayonnement ultraviolet, endommage leur ADN selon YEJIN GE et al. (10 mars 2020, The aging skin microenvironment dictates stem cell behavior, PNAS, Vol. 117, p. 5339-5350). Ces dommages altèrent leur capacité d'auto-renouvellement et de différenciation, conduisant à une diminution du pool de cellules souches et finalement au vieillissement de la peau.

L'objectif de l'étude est d'évaluer l'effet de l'hydrolysat de protéines *peregrina* selon l'invention sur la protection des cellules souches épidermiques contre l'irradiation UVB.

Protocole : Des cellules de kératinocytes humains ont été obtenues auprès d'un donneur de 62 ans. Pour réaliser les expériences, les kératinocytes ont été cultivés en monocouche jusqu'à atteignant 80% de confluence.

La culture cellulaire a ensuite été enrichie en cellules souches épidermiques en suivant la méthode décrite par GOODELL, M. et al. (1996, Hoescht 33342 HSC staining and stem cell purification protocol Journal of Experimental Medicine 183, p. 1797-806).

Produit de référence : La quercétine à 1 µM a été utilisée comme produit de référence dans cette étude. La quercétine a été achetée auprès de Sigma Aldrich.

Les cellules ont été pré-incubées pendant 24 heures en absence (« Contrôle ») ou en présence du produit de référence ou d'une concentration croissante du composé à tester. À la fin de la période de pré-incubation, les cellules ont été irradiées par UVB (30mJ / cm2) puis incubées pendant 8 jours à 37 ° C en absence (témoin) ou en présence de produit de référence ou en augmentant la concentration du composé à tester.

« Hydrolysat de protéines *peregrina »* : 0.01; 0.05 and 0.15% (v/v).

### Préparation du composé d'essai :

Le composé d'essai « Hydrolysat de protéines *peregrina »* a été dilué directement dans le milieu d'incubation afin d'atteindre les différentes concentrations décrites ci-dessus.

À la fin de la période d'incubation, la viabilité cellulaire a été mesurée en utilisant du bleu Alamar, un indicateur de viabilité non cytotoxique basé sur la réduction de la rézazurine par les mitochondries. Chaque condition expérimentale a été réalisée en triplicate (n = 3).

Les résultats sont présentés ci-dessous en pourcentage de viabilité par rapport à la condition expérimentale « Contrôle sans UVB » (moyenne +/- S.D). Le niveau de signification entre « Contrôle sans UVB » et « Contrôle avec UVB » a été évalué à l'aide d'un test de Student (p <0.05).

**[Tableau 10]**

| | Concentration de l'hydrolysat | Croissance cellulaire versus contrôle (%) | I Protection des cellules souches versus contrôle (%) |
|---|---|---|---|
| Hydrolysat de protéines *peregrina* | 0.15% | ca. -9.2 | NS |
| | 0.05% | -3.6 | +30.50 |
| | 0.01% | ca. -6 | +15.50 |

Conclusion : L'hydrolysat de protéines *peregrina* peut protéger de manière significative les cellules souches de la peau humaine soumises à un stress cellulaire (UV). Les cellules souches sont des cellules avec matériel d'ADN préservé et jeune. Elles sont à l'origine de la régénération tissulaire, du retour à un état jeune et sain. La protection des cellules souches est corrélée à la capacité de préserver le matériel ADN. L'hydrolysat de protéines *peregrina* selon l'invention maintient l'intégrité des cellules souches ; il est ainsi impliqué dans la conservation de l'ADN.

### Exemple 6 : Effet de l'hydrolysat de protéines peregrina selon l'invention pour préserver l'ADN

La dynamique de la longueur des télomères est très importante pour la régulation de la durée de vie réplicative dans les cellules, en particulier chez les espèces à longue durée de vie. Le raccourcissement des télomères et l'activité de la télomérase sont des facteurs importants dans le vieillissement et la tumorigenèse (SHAY, J. W. & WRIGHT, W. E., 2005, Senescence an Immortalization: Role of Telomeres and Telomerase, Carcinogenesis 26(5), p. 867-874). Les télomères sont des séquences nucléotidiques complexes qui recouvrent l'extrémité des chromosomes de la dégradation, de la fusion-recombinaison indésirable, de l'activation inappropriée de la réponse aux dommages de l'ADN. Ils jouent également un rôle essentiel dans la division cellulaire et la stabilité des chromosomes. Il existe de plus en plus de preuves que la stabilité des télomères et leur longueur moyenne peut être affectée par des stress notamment environnementaux ou maladies sous influence environnementale (VALDES, A.L. et al., July 2005, Obesity, cigarette smoking and telomere length in women, Research Letters,366(9486), p. 662-664 ; PHILLIPS A.C. et al., 2013, Do symptoms of depression predict telomere length? Evidence from the West of Scotland Twenty-07 Study, Psychosomatic Medicine, 75(3), p. 288-296; SHIN, D. et al. May 2019, Effects of inflammation and depression on telomere length in young adults in the United States, Journal of Clinical Med 2019, 8(5), p. 711; SALIQUES, S. et al., October 2010, Telomer length and cardiovascular disease, Archives of Cardiovascular Diseases, 103(8-9), p. 454-459). Ainsi, les télomères extrêmement courts ont été associés à des maladies neurodégénératives, cardiovasculaires (MCV) et au risque de cancer.

La télomérase est une ribonucléoprotéine qui catalyse l'addition de répétitions télomériques aux extrémités des télomères. Les télomères sont de longs tronçons de séquences répétées qui coiffent les extrémités des chromosomes et sont censés stabiliser le chromosome. Chez l'homme, les télomères ont généralement une longueur de 7 à 10 kb et comprennent plusieurs répétitions de la séquence - TTAGGG-.

La télomérase n'est pas exprimée dans la plupart des cellules adultes et la longueur des télomères diminue avec les cycles de réplication successifs. Après un certain nombre de cycles de réplication, le raccourcissement progressif des télomères fait entrer les cellules dans une phase de crise télomérique, qui à son tour conduit à la sénescence cellulaire. Certaines maladies sont associées à une perte télomérique rapide, entraînant une sénescence cellulaire prématurée. Il a été démontré que l'expression du gène codant pour la protéine de télomérase humaine dans les cellules humaines (BLASCO M., 2007, Telomere Length, Stem Cells and Aging, Nature Chemical Biology, 3(10), p. 640-649) confère un phénotype de qualité constante, probablement en contournant la voie de sénescence naturelle des cellules. De plus, il a été démontré dans l'étude précitée que l'expression du gène de la télomérase dans les cellules vieillissantes avec de courts télomères produit une augmentation de la longueur des télomères et restaure un phénotype généralement associé aux cellules plus jeunes.

L'objectif de cette étude est d'évaluer l'effet du composé dénommé « hydrolysat de protéines *peregrina »* sur le raccourcissement des télomères dans un modèle composé de fibroblastes humains normaux en culture monocouche. Il est bien connu que le télomère correspond à une horloge biologique. La longueur des télomères diminue progressivement avec les divisions cellulaires, entraînant finalement une cellule incapable de réplication. La mesure de la longueur des télomères a été effectuée en utilisant la PCR quantitative et en comparaison avec la longueur des télomères entre les cellules aux passages 2 et 5.

Protocole : Des cellules de fibroblastes humains ont été obtenues auprès d'un donneur de 44 ans. Pour effectuer les expériences, des cellules ont été utilisées au passage 2 et 5. Les fibroblastes ont été cultivés pendant 3 passages consécutifs en absence (contrôle) ou en présence d'une concentration croissante à tester de l'hydrolysat de protéines *peregrina:* 0.01; 0.1 et 0.5% (v/v).

Préparation du composé d'essai : Le composé d'essai « hydrolysat de protéines *peregrina »* a été dilué directement dans le milieu d'incubation afin d'atteindre les différentes concentrations décrites ci-dessus.

A la fin de l'incubation, les cellules ont été trypsinisées. L'ADN a été extrait des cellules à l'aide d'un kit d'extraction d'ADN dédié. L'ADN a été quantifié par nanodrop.

La longueur des télomères a été mesurée par PCR quantitative (q-PCR). Pour chaque échantillon, la variation de la longueur des télomères a été mesurée par quantification relative en utilisant le gène SCR (référence à copie unique) comme gène de référence. Pour chaque échantillon, une q-PCR est effectuée en utilisant un jeu d'amorces de télomères qui reconnaît et amplifie les séquences de télomères et une seconde q-PCR est effectuée en utilisant le jeu d'amorces SCR qui reconnaît et amplifie une région de 100 pb sur le chromosome 17 humain et sert de référence pour la normalisation des données.

Les résultats sont exprimés en unités relatives correspondant à la longueur des télomères par rapport aux cellules au passage 2 (moyenne ± S.D.). Le niveau de signification versus « Contrôle » aux passages 2 et 5 a été évalué à l'aide d'un test de Student (*: p <0,05). Le niveau de signification entre « contrôle » et « composé d'essai » a été évalué indépendamment pour chaque produit par une analyse de variance à un facteur (ANOVA unidirectionnelle) suivie d'un test Holm-Sidak (*: p <0,05).

**[Tableau 11]**

| | Concentration | Croissance cellulaire versus contrôle (%) | Longueur des télomères versus contrôle (%) |
|---|---|---|---|
| Hydrolysat de protéines | 0.5% | +1.9 | +16.60 |
| | 0.1% | ca. 0 | +15.10 |
| | 0.05% | -0.6 | +8.90 |

Résultats : Dans nos conditions expérimentales, l'hydrolysat de protéines *peregrina* testé à 0.05%, 0.1% et 0.5% (v / v), a significativement diminué le raccourcissement des télomères des fibroblastes humains normaux.

Le raccourcissement des télomères présente une inhibition (en comparaison avec le contrôle) à 0,05% (v / v) de + 8,9% (p <0,05) ; à 0,1% (v / v) de + 15,1% (p <0,01) et à 0,5% (v / v) de + 16,6% (p <0,01). Conclusion : Dans un contexte de multiplication ou de division normale des cellules humaines, l'hydrolysat de protéines *peregrina* selon l'invention démontre une capacité à augmenter significativement la longueur des télomères. Les télomères sont des bouchons impliqués dans la protection du matériel ADN ; l'augmentation de la longueur des télomères est corrélée à la capacité de préserver le matériel d'ADN. L'hydrolysat de protéines *peregrina* peut augmenter la longueur des télomères. Cet hydrolysat est donc impliqué dans la préservation du matériel génétique humain (ADN).

Étude complémentaire de l'évaluation des extraits P1, P2, P3 et du complexe P1+P2 décrits à l'exemple 12 sur leur capacité à protéger l'ADN en augmentant la longueur des télomères après plusieurs divisions cellulaires.

**[Tableau 12]**

| | **Passage 2** | **Passage 5** | | | |
|---|---|---|---|---|---|
| | Témoin | Témoin | Extrait P1 (%; v/v) | | |
| | | | 0.01% | 0.3% | 1% |
| Variation de la longueur des télomères (changement de taille par rapport au premier passage) | 1.08 | 0.56 | 0.57 | 0.65 | 0.74 |
| | 0.86 | 0.58 | 0.61 | 0.64 | 0.77 |
| | 1.07 | 0.54 | 0.62 | 0.71 | 0.70 |
| Moyenne | 1.00 | 0.56 | 0.60* | 0.67** | 0.74*** |
| Ecart Type | 0.12 | 0.02 | 0.03 | 0.04 | 0.04 |
| **Longueur Télomères (en % du Témoin)** | 100.0 | 55.8 | 59.8 | 66.6 | 73.3 |
| **% d'allongement des télomères par rapport au témoin** | | 0 | 9.1 | 24.3 | 39.6 |

| | | | | | |
|---|---|---|---|---|---|
| *Significativité statistique (p>0.05) **: Significativité statistique (p<0.01) ***: Significativité statistique (p<0.001) | | | | | |

L'extrait P1 selon l'invention après 3 divisions cellulaires dès la concentration de 0,3% augmente de 24,3% la taille des télomères sur un modèle de culture de cellules humaines normales (fibroblastes). A la concentration de 1%, l'extrait P1 augmente dans les mêmes conditions la taille des télomères de 39,6%.

**[Tableau 13]**

| | **Passage 2** | **Passage 5** | | | |
|---|---|---|---|---|---|
| | Témoin | Témoin | Extrait P2 (%; v/v) | | |
| | | | 0.01% | 0.3% | 1% |
| Variation de la longueur des télomères (changement de taille par rapport au premier passage) | 1.08 | 0.56 | 0.53 | 0.61 | 0.66 |
| | 0.86 | 0.58 | 0.70 | 0.71 | 0.68 |
| | 1.07 | 0.54 | 0.74 | 0.68 | 0.92 |
| Moyenne | 1.00 | 0.56 | 0.66* | 0.67* | 0.75* |
| Ecart Type | 0.12 | 0.02 | 0.11 | 0.05 | 0.14 |
| **Longueur Télomères (en % du Témoin)** | 100.0 | 55.8 | 65.3 | 66.3 | 75.0 |
| **% d'allongement des télomères par rapport au témoin** | | 0 | 21.4 | 23.7 | 43.5 |

| | | | | | |
|---|---|---|---|---|---|
| *Significativité statistique (p>0.05) | | | | | |

L'extrait P2 selon l'invention après 3 divisions cellulaires sur un modèle de culture de cellules humaines normales (fibroblastes) présente une tendance non confirmée statistiquement à l'allongement des télomères pour chaque concentration testée.

**[Tableau 14]**

| | **Passage 2** | **Passage 5** | | | |
|---|---|---|---|---|---|
| | Témoin | Témoin | Extrait P3 (%; v/v) | | |
| | | | 0.01% | 0.3% | 1% |
| Variation de la longueur des télomères (changement de taille par rapport au premier passage) | 1.08 | 0.56 | 0.51 | 0.46 | 0.50 |
| | 0.86 | 0.58 | 0.52 | 0.54 | 0.55 |
| | 1.07 | 0.54 | 0.64 | 0.61 | 0.61 |
| Moyenne | 1.00 | 0.56 | 0.56* | 0.54* | 0.55* |
| Ecart Type | 0.12 | 0.02 | 0.07 | 0.07 | 0.06 |
| **Longueur Télomères (en % du Témoin)** | 100.0 | 55.8 | 55.4 | 53.6 | 55.2 |
| **% d'allongement des télomères par rapport au témoin** | | 0 | -0.8 | -5.0 | -1.3 |

| | | | | | |
|---|---|---|---|---|---|
| *Significativité statistique (p>0.05) | | | | | |

L'extrait P3 selon l'invention après 3 divisions cellulaires sur un modèle de culture de cellules humaines normales (fibroblastes) ne présente aucune capacité à promouvoir l'allongement des télomères pour chaque concentration testée.

**[Tableau 15]**

| | **Passage 2** | **Passage 5** | | | |
|---|---|---|---|---|---|
| | Témoin | Témoin | Extrait P1+P2 (%; v/v) | | |
| | | | 0.01% | 0.3% | 1% |
| Variation de la longueur des télomères (changement de taille par rapport au premier passage) | 1.08 | 0.56 | 0.61 | 0.72 | 0.76 |
| | 0.86 | 0.58 | 0.84 | 0.67 | 0.71 |
| | 1.07 | 0.54 | 0.63 | 0.74 | 0.86 |
| Moyenne | 1.00 | 0.56** | 0.70* | 0.71* | 0.78** |
| Ecart Type | 0.12 | 0.02 | 0.13 | 0.04 | 0.08 |
| **Longueur Télomères (en % du Témoin)** | 100.0 | 55.8 | 69.3 | 70.5 | 77.5 |
| **allongement des télomères par rapport au témoin** | | 0 | 30.5 | 33.3 | 49.1 |

| | | | | | |
|---|---|---|---|---|---|
| *Significativité statistique (p>0.05) **: Significativité statistique (p<0.01) | | | | | |

Le mélange des extraits P1 et P2 (50/50 en volume) selon l'invention après 3 divisions cellulaires à la concentration de 1% (soit 0,5% de chaque extrait) augmente de 49,1% la taille des télomères sur un modèle de culture de cellules humaines normales (fibroblastes). Ce score n'ayant pas été atteint par les extraits individuels, un effet synergique est ainsi démontré en mélangeant l'extrait P1 et P2.

Conclusion : Les composés nommés « Extrait P1 » et « Extrait P1 + P2 » diminuent significativement le raccourcissement des télomères survenant après 3 passages cellulaires consécutifs. Une synergie est confirmée en combinant P1 et P2.

### Exemple 7: Effet de l'hydrolysat de protéines peregrina selon l'invention pour stimuler la protéine ZAG

L'alpha-2-glycoprotéine de zinc (ZAG) est une glycoprotéine plasmatique qui tire son nom de sa mobilité électrophorétique et de sa capacité à être précipité par les sels de Zn. La ZAG fait partie de la superfamille des gènes d'immunoglobulines et possède une structure tridimensionnelle hautement homologue aux molécules du CMH de classe I et II. La ZAG a été détectée immunohistochimiquement dans les cellules épithéliales sécrétoires normales du sein, de la prostate et du foie, dans les glandes salivaires, bronchiques, gastro-intestinales et sudoripares et dans les épithéliums stratifiés normaux, y compris l'épiderme. L'ARNm de ZAG reste uniformément distribué dans différents types de cellules (FREIJE, J. P. et al., 1991, Human Zn-α2-Glycoprotein cDNA Cloning and Expression Analysis in Benign and Malignant Breast Tissues, FEBS Letters 290 (1-2), p. 247-249). Du fait de sa production par l'épithélium sécrétoire, la ZAG est présente dans la plupart des sécrétions corporelles et constitue respectivement 2,5% des protéines de la salive et 30% des fluides séminaux. Il a été décrit que les taux plasmatiques ou sériques de ZAG varient avec l'âge, avec des valeurs rapportées allant de 0,9 à 3,5 mg / dl (fœtus) à 7,8 à 12,1 mg / dl (jeunes adultes) (JIRKA, M. et al., 1974, The Zn-alpha 2-Glycoprotein Level in Human Serum During Ontogenesis. Clinica Chimica Acta 56, p. 31-33 ; JIRKA, M. et al., 1978, Human Serum Zn-α2-Glycoprotein in Amniotic Fluid, Clinica Chimica Acta 85, p. 107-110). La ZAG s'accumule dans les liquides kystiques mammaires jusqu'à une concentration plasmatique de 30 à 50 fois (BUNDRED et al., 1987, An Immunohistochemical Study of the Tissue Distribution of the Breast Cyst Fluid Protein, Zinc Alpha2-Glycoprotein, Histopathology 11, p. 603-610 ; DIEZ-ITZA, I. et al., 1993, Zn-α2-Glycoprotein Levels in Breast Cancer Cytosols and Correlation with Clinical, Histological, and Biochemical Parameters, European Journal of Cancer 29A, p. 1256-1260) et est surexprimée dans 40 à 50% des carcinomes mammaires. Il a récemment été démontré (SUSAN, M. el al., Zinc-alpha2-glycoprotein Expression as a Predictor of Metastatic Prostate Cancer Following Radical Prostatectomy, 2006, Journal of the National Cancer Institute, Volume 98(19), p. 1420-1424) que la ZAG est produite en quantité par la plupart des carcinomes de la prostate, ce qui entraîne une élévation des taux sériques de ZAG chez les patients atteints de cancer de la prostate dans les carcinomes basocellulaires. L'objectif de cette étude est d'évaluer la capacité de l'hydrolysat de protéines *peregrina* à stimuler la ZAG.

Protocole : Les kératinocytes humains normaux sont isolés à partir de prépuces et mis en culture dans les plaques 24 et 96 puits selon les procédures internes.

Il s'agit de laisser agir les échantillons à des concentrations définies sur les kératinocytes à 80% de confluence pendant 48 heures, puis de quantifier les ZAG à l'aide du kit ELISA dans les surnageants cellulaires.

Un test préalable de viabilité est réalisé pour définir les doses non-toxiques à utiliser lors de dosage des ZAG. Le contrôle négatif est réalisé à l'aide des cellules en milieu de culture sans traitement. Le contrôle positif pour le test de viabilité est le SDS à 0.5%.

Toutes les conditions sont préparées dans des milieux de culture, et les cellules sont ensuite incubées à 36.5°C / 5% CO2 pendant 24 heures pour le test de viabilité et 48 heures pour le dosage des ZAG.
- Application des solutions à tester sur les kératinocytes :
   - Les produits à tester sont mis en contact avec les kératinocytes en subconfluence dans les plaques 24 et 96 puits.
   - Pour chaque concentration, le test est réalisé sur 3 puits.
   - Les plaques sont incubées pendant 24 heures et 48 heures à 36.5°C / 5% CO2.
- Test de viabilité :
   - La viabilité cellulaire est évaluée par la méthode MTT sur les cellules après incubation avec les produits.
   - Après 24 et 48 heures d'incubation, les puits prévus sont rincés 1 fois avec 200µL de PBS.
   - Dans chaque puits 50 µl de solution de MTT à 0.5 mg/ml sont ajoutés : incubation pendant 3 heures à 36.5°C / 5% CO2.
   - Dans chaque puits 100 µl d'isopropanol sont ajoutés.
   - Après homogénéisation, une lecture de l'absorbance est réalisée à 550 nm.
   - Pour chaque condition, le rapport de la moyenne des densités optiques des cellules sur la moyenne des densités optiques des contrôles négatifs déterminera le taux de viabilité.
- Dosage des protéines ZAG :
   - Après 48 heures d'incubation, tous les surnageants sont récupérés et conservés à -20°C pour les dosages.
   - Le dosage est réalisé à l'aide d'un kit ELISA. Les résultats sont donnés ci-dessous.

**[Tableau 16]**

| | Concentration | Croissance cellulaire versus contrôle (%) | ZAG versus contrôle (%) | ZAG versus contrôle (ng/ml) |
|---|---|---|---|---|
| Hydrolysat de protéines | 2% | -17.88 | +337.80 | 0.390 |
| | 1% | -12.54 | +195.73 | 0.157 |
| | 0.10% | -3.88 | +151.83 | 0.085 |

Conclusion : L'hydrolysat de protéines *peregrina* peut augmenter de manière significative la production de ZAG, avec une bonne dépendance à la dose et une faible toxicité sur les cellules humaines. Il a de ce fait un effet anti-fibrotique et un effet anti-inflammatoire fondé sur sa capacité à augmenter significativement la ZAG.

Une étude complémentaire de la stimulation de la ZAG à partir des bandes protéiques identifiées à l'exemple 12 est décrite ci-après sur kératinocytes humains normaux.

**[Tableau 17]**

| | **Témoin** | **Extrait P1 (%, v/v)** | | |
|---|---|---|---|---|
| | | **0.2** | **1** | **5** |
| ZAG (pg/µg de protéines) | 2.8 | 4.1 | 4.3 | 2.3 |
| | 3.0 | 6.0 | 3.9 | 1.1 |
| | 2.0 | 6.1 | 3.4 | 2.4 |
| **Moyenne** | **2.6** | **5.4**** | **3.9*** | **1.9*** |
| Ecart Type | 0.5 | 1.1 | 0.5 | 0.7 |
| **% du Témoin** | **100.0** | **207.1** | **147.3** | **73.5** |

| | | | | |
|---|---|---|---|---|
| *Significativité statistique (p>0.05) **: Significativité statistique (p<0.01) | | | | |

L'extrait P1 dès 0,2% augmente significativement la production de ZAG de plus de 107%.

**[Tableau 18]**

| | **Témoin** | **Extrait P2 (%, v/v)** | | |
|---|---|---|---|---|
| | | **0.2** | **1** | **5** |
| ZAG (pg/µg de protéines) | 2.8 | 6.2 | 4.3 | 2.6 |
| | 3.0 | 4.2 | 4.4 | 2.7 |
| | 2.0 | 3.7 | 4.5 | 2.3 |
| **Moyenne** | **2.6** | **4.7**** | **4.4**** | **2.5*** |
| Ecart Type | 0.5 | 1.4 | 0.1 | 0.2 |
| **% du Témoin** | **100.0** | **180.1** | **167.5** | **95.6** |

| | | | | |
|---|---|---|---|---|
| *Significativité statistique (p>0.05) **: Significativité statistique (p<0.05) | | | | |

L'extrait P2 dès 0,2% augmente significativement la production de ZAG de plus de 80%.

**[Tableau 19]**

| | **Témoin** | **Extrait P3 (%, v/v)** | | |
|---|---|---|---|---|
| | | **0.2** | **1** | **5** |
| ZAG (pg/µg de protéines) | 2.8 | 2.1 | 2.3 | 2.3 |
| | 3.0 | 1.9 | 2.7 | 1.8 |
| | 2.0 | 2.2 | 2.1 | 3.3 |
| **Moyenne** | **2.6** | **2.1*** | **2.4*** | **2.5*** |
| Ecart Type | 0.5 | 0.2 | 0.3 | 0.8 |
| **% du Témoin** | **100.0** | **78.5** | **90.0** | **95.2** |

| | | | | |
|---|---|---|---|---|
| *Significativité statistique (p>0.05) | | | | |

L'extrait P3 n'a pas la capacité d'influencer de manière significative la production de ZAG dans ce modèle d'étude.

**[Tableau 20]**

| | **Témoin** | **Extrait P1+P2 (50/50) (%, v/v)** | | |
|---|---|---|---|---|
| | | **0.2** | **1** | **5** |
| ZAG (pg/µg de protéines) | 2.8 | 3.9 | 4.2 | 1.7 |
| | 3.0 | 3.7 | 3.5 | 2.6 |
| | 2.0 | 3.5 | 2.8 | 2.6 |
| **Moyenne** | **2.6** | **3.7*** | **3.5*** | **2.3*** |
| Ecart Type | 0.5 | 0.2 | 0.7 | 0.5 |
| **% du Témoin** | **100.0** | **140.6** | **132.9** | **87.8** |

| | | | | |
|---|---|---|---|---|
| *Significativité statistique (p>0.05) | | | | |

La combinaison des extraits P1 et P2 montre une tendance à l'activation de la production de ZAG, mais cette tendance n'est pas significative d'un point de vue statistique. Il n'y a donc pas d'effet synergique dans la combinaison des extraits P1 et P2.

Les composés dénommés « Extrait P1 » et « Extrait P2 » augmentent significativement la ZAG libérée en milieu de culture par les kératinocytes normaux humains en culture monocouche, mais aucune synergie n'est constatée en combinant P1 et P2.

Conclusion : Dans l'extrait hydrolysé selon l'invention, l'extrait P1 est l'extrait le plus performant pour augmenter la ZAG. Il a de ce fait un effet anti-fibrotique et un effet anti-inflammatoire dès la dose de 0,2%.

### Exemple 8 : Effet de l'hydrolysat de protéines peregrina selon l'invention sur la modulation du dosage de DKK1 et DKK3

L'implication des interactions entre les mélanocytes et les fibroblastes dans la régulation de la mélanogenèse est bien connue et a été étudiée de manière intensive. Bien que ces interactions ne soient pas encore parfaitement comprises, elles sont à l'origine de la "blancheur" des zones palmo-plantaires et sont désormais utilisées en dermatologie pour le développement de produits dépigmentants. Yamaguchi et collègues (YAMAGUCHI Y. et al., 2004, Mesenchymal-Epithelial Interactions in the Skin: Increased Expression of Dickkopf by Palmoplantar Fibroblasts Inhibits Melanocyte Growth and Differentiation, Journal of Cell Biology 165(2), p. 275-285) ont démontré qu'un messager soluble produit par les fibroblastes des régions palmo-plantaires, était capable de modifier le programme de différenciation des mélanocytes de ces régions, conduisant à une diminution de la production de mélanine. Ce messager a été identifié par l'équipe comme une protéine nommée Dikkopf-1 (DKK-1).

Les voies de signalisation utilisées par DKK-1 pour produire ces résultats sont bien identifiées aujourd'hui. Grâce à son action antagoniste sur le récepteur Wnt, DKK-1 est en effet capable de court-circuiter les voies de signalisation intracellulaires activées par la β-caténine, généralement responsables de la régulation des gènes impliqués dans la mélanogenèse. Yamaguchi et collègues (cf. *supra*) ont également démontré que DKK-3, une molécule comme DKK-1 mais sans effet sur le récepteur Wnt, pourrait jouer un rôle régulateur sur l'effet de DKK-1. En fait, plus la quantité de DKK-3 au voisinage de ce récepteur à Wnt augmente, plus les interactions entre DKK-1 et ce récepteur sont faibles. L'augmentation de DKK3 réduit les effets inhibiteurs de DKK-1 sur la mélanogenèse. Les travaux de Yamaguchi et collègues (cf. *supra*) suggèrent encore que l'identification d'agents ayant une influence sur le rapport DKK1 / DKK3 dans des cultures de fibroblastes dermiques humains normaux d'origine non palmo-plantaire permettrait de contrôler la production de mélanine à partir de mélanocytes humains normaux non palmo-plantaires.

L'objectif de cette étude est d'évaluer l'effet de l'hydrolysat de protéines *peregrina* sur la synthèse et la libération de DKK-1 dans un modèle composé de fibroblastes humains normaux en culture monocouche.

Protocole : Les cellules de fibroblastes humains sont obtenues auprès d'un donneur âgé de 68 ans. Pour réaliser les expériences, les fibroblastes sont cultivés en monocouche jusqu'à atteindre la confluence. La dexaméthasone à 100 nm a été utilisée comme inducteur de référence de la synthèse et de la libération de DKK-1. Les disques cutanés ont été incubés pendant 48 heures en absence (contrôle) ou en présence de produit de référence ou de produit à tester : « Hydrolysat de protéines *peregrina »* : 0.01; 0.1 et 0.5% (v/v).

À la fin de l'incubation, les milieux d'incubation ont été retirés pour effectuer la mesure de libération de DKK-1.

Le composé d'essai « hydrolysat de protéines *peregrina »* a été dilué directement dans le milieu d'incubation afin d'atteindre les différentes concentrations décrites ci-dessus.

À la fin de la période d'incubation de 48 heures, le DKK-1 libéré dans les milieux d'incubation a été quantifié à l'aide d'un kit ELISA sensible et spécifique.

À la fin de la période d'incubation, les protéines contenues dans les lysats cellulaires ont été quantifiées à l'aide d'une méthode spectro-colorimétrique (méthode Bradford).

Les résultats sont exprimés en ng de DKK-1 par mg de protéines (moyenne ± S.D.).

Le niveau de signification entre le « contrôle » et le « produit de référence » a été évalué à l'aide d'un test de Student (p <0,05).

Le niveau de signification entre le « Contrôle » et le « produit d'essai » a été évalué par une analyse de variance à un facteur (ANOVA unidirectionnelle) suivie d'un test de Holm-Sidak (p <0,05).

Dans nos conditions expérimentales, le produit de référence nommé « Dexaméthasone », testé à 100 nm, a significativement augmenté le DKK-1 libéré de 181,8% (p <0,01) par rapport à « Contrôle ». Les résultats sur la modulation du dosage de DKK1 sont donnés ci-dessous.

**[Tableau 21]**

| | Concentration | Croissance cellulaire versus contrôle (%) | DKK1 versus contrôle (%) |
|---|---|---|---|
| Hydrolysat de protéines | 0,5% | +1,9 | +131,50 |
| | 0,1% | ca. 0 | +32,30 |
| | 0,05% | -0,6 | +26,10 |

L'étude montre que l'hydrolysat selon l'invention augmente significativement le taux de DKK1 à la dose de 0,05% avec 26,1% d'augmentation par rapport au taux basal et à la concentration de 0,5% de l'extrait selon l'invention il est observé une augmentation de 131,5% du taux basa

Le but de cette étude est d'évaluer l'effet de l'hydrolysat de protéines *peregrina* sur la synthèse et la libération de DKK-3 dans un modèle composé de fibroblastes humains normaux en culture monocouche. Les cellules de fibroblastes humains ont été obtenues auprès d'un donneur âgé de 68 ans. Pour réaliser les expériences, les fibroblastes ont été cultivés en monocouche jusqu'à atteindre la confluence. Les cellules de fibroblastes humains ont été obtenues d'un donneur de 68 ans. Pour réaliser les expériences, les fibroblastes ont été cultivés en monocouche jusqu'à atteindre la confluence.

À la fin de la période d'incubation de 48 heures, le DKK-3 libéré dans les milieux d'incubation a été quantifié à l'aide d'un kit ELISA sensible et spécifique.

À la fin de la période d'incubation, les protéines contenues dans les lysats cellulaires ont été quantifiées à l'aide d'une méthode spectro-colorimétrique (méthode Bradford).

Les résultats sont exprimés en ng de DKK-3 par mg de protéines (moyenne ± S.D.). Le niveau de signification entre le « Contrôle » et le « produit de référence » a été évalué à l'aide d'un test de Student (*: p <0.05).

Le niveau de signification entre le « Contrôle » et l'hydrolysat de protéines *peregrina* a été évalué par une analyse de variance à un facteur (ANOVA unidirectionnelle) suivie d'un test de Holm-Sidak (*: p <0.05). Les résultats sur la modulation du dosage de DKK3 sont donnés ci-après.

**[Tableau 22]**

| | Concentration | Croissance cellulaire versus contrôle (%) | DKK3 versus contrôle (%) |
|---|---|---|---|
| Hydrolysat de protéines | 0.5% | 101.9 | -20.7 |
| | 0.1% | ca. 100.0 | -8 |
| | 0.05% | 99.4 | -3.9 |

Conclusion : L'hydrolysat de protéines *peregrina* selon l'invention à 0,5% présente une inhibition notable de l'ordre de 21% de la DKK3 par rapport au taux basal. L'hydrolysat de protéines *peregrina* selon l'invention a une forte capacité à gérer les gènes impliqués dans la différentiation cellulaire grâce au principe d'inhibition palmo-plantaire (voie de signalisation béta-caténine), par sa capacité à augmenter considérablement DKK1 et diminuer significativement DKK3, ce qui augmente le ratio DKK1/DKK3.

Étude complémentaire de l'évaluation de P1, P2, P3 et du complexe P1+P2 décrits à l'exemple 10 sur la production de DKK1 : Modèle de test cellulaire sur Fibroblastes humains normaux.

**[Tableau 23]**

| | **Témoin** | **Extrait P1 (%; v/v)** | | |
|---|---|---|---|---|
| | | **0.01** | **0.3** | **1** |
| DKK-1 (ng/mg of proteins) | 306.7 | 334.8 | 399.5 | 372.9 |
| | 294.9 | 293.1 | 347.9 | 392.0 |
| | 298.6 | 367.6 | 392.4 | 385.1 |
| **Moyenne** | **300.1** | **331.9*** | **379.9**** | **383.3**** |
| Ecart Type | 6.1 | 37.3 | 28.0 | 9.7 |
| **% du Témoin** | **100.0** | **110.6** | **126.6** | **127.8** |

| | | | | |
|---|---|---|---|---|
| *Significativité statistique (p>0.05) **: Significativité statistique (p<0.01) | | | | |

L'extrait P1 augmente significativement la production de DKK1 de 26,6% dès la dose de 0,3%, et de 27,8% à la dose de 1%.

**[Tableau 24]**

| | **Témoin** | **Extrait P2 (%; v/v)** | | |
|---|---|---|---|---|
| | | **0.01** | **0.3** | **1** |
| DKK-1 (ng/mg de protéines) | 306.7 | 291.5 | 292.6 | 410.8 |
| | 294.9 | 346.5 | 275.4 | 367.0 |
| | 298.6 | 277.4 | 354.9 | 344.4 |
| **Moyenne** | **300.1** | **305.2*** | **307.6*** | **374.1*** |
| Ecart Type | 6.1 | 36.5 | 41.8 | 33.8 |
| **% du Témoin** | **100.0** | **101.7** | **102.5** | **124.7** |

| | | | | |
|---|---|---|---|---|
| *Significativité statistique (p>0.05) | | | | |

L'extrait P2 n'augmente pas statistiquement significativement la production de DKK1 à chacune des doses expérimentées.

**[Tableau 25]**

| | **Témoin** | **Extrait P3 (%; v/v)** | | |
|---|---|---|---|---|
| | | **0.01** | **0.3** | **1** |
| DKK-1 (ng/mg de protéines) | 306.7 | 359.2 | 387.0 | 224.7 |
| | 294.9 | 371.8 | 263.7 | 283.0 |
| | 298.6 | 293.4 | 287.3 | 288.6 |
| **Moyenne** | **300.1** | **341.5*** | **312.7*** | **265.5*** |
| Ecart Type | 6.1 | 42.1 | 65.5 | 35.4 |
| **% du Témoin** | **100.0** | **113.8** | **104.2** | **88.5** |

| | | | | |
|---|---|---|---|---|
| *Significativité statistique (p>0.05) | | | | |

L'extrait P3 n'augmente pas statistiquement significativement la production de DKK1 à chacune des doses expérimentées.

**[Tableau 26]**

| | **Témoin** | **Extrait P1+P2 50/50(%; v/v)** | | |
|---|---|---|---|---|
| | | **0.01** | **0.3** | **1** |
| DKK-1 (ng/mg of proteins) | 306.7 | 305.9 | 366.6 | 389.8 |
| | 294.9 | 311.1 | 357.1 | 479.5 |
| | 298.6 | 346.8 | 308.1 | 384.2 |
| **Moyenne** | **300.1** | **321.3*** | **343.9*** | **417.8**** |
| Ecart Type | 6.1 | 22.2 | 31.4 | 53.5 |
| **% du Témoin** | **100.0** | **107.1** | **114.6** | **139.2** |

| | | | | |
|---|---|---|---|---|
| *Significativité statistique (p>0.05) **: Significativité statistique (p<0.01) | | | | |

La combinaison des extraits P1 et P2 a la dose de 1% (soit 0,5% pour chacun des extraits) augmente de plus de 39% et de manière significative la production de DKK1. Ce score est supérieur à celui atteint avec l'extrait seul de P1 ou de P2 ; ceci traduit donc un effet synergique de la combinaison des deux extraits P1 et P2.

Conclusion : Les composés dénommés « Extrait P1 » et « Extrait P1 + P2 » ont significativement augmenté la DKK-1 libérée dans le milieu de culture par les fibroblastes normaux humains en culture monocouche. Une synergie est confirmée en mélangeant P1 et P2.

### Conclusion des activités précédentes de l'hydrolysat de protéines selon l'invention :

L'activité la plus remarquable de l'hydrolysat de protéines *peregrina* démontrée dans les tests *in cellulo* est son action épigénétique avec sa capacité à ralentir très significativement le processus de raccourcissement des télomères après les divisions cellulaires. L'hydrolysat est un protecteur cellulaire, plus particulièrement des cellules souches, ce qui en fait un très bon régénérateur cellulaire et tissulaire. Ces propriétés offrent une très forte protection de l'ADN et de son matériel génétique. L'hydrolysat de protéines *peregrina* est également un puissant modulateur de la production de ZAG et d'endothéline de type I, avec un effet anti-fibrotique et anti-inflammatoire.

### Exemple 9 : Evaluation de l'effet de l'hydrolysat de protéines de tourteau délipidé de peregrina sur l'Angiotensine convertase 2 (ACE2) - étude acellulaire de l'effet inhibiteur.

L'Angiotensine convertase 2 est notamment impliquée dans l'infection intracellulaire du COVID19 après l'activation des spikes protéines par d'autres convertases et plus particulièrement la furine, voir PETER BRADDING et al. (ACE, TMPRSS2, and furin gene expression in the airways of people with asthma - implications of COVID-19, JOURNAL ALLERGY CLINICAL IMMUNOLOGY, July 2020, n°146(1), p. 206-211 ).

**[Tableau 27]**

| **Echantillons** | | **Moyenne** | **Moy-blanc** | **% activité enzymatique** | **% inhibition** |
|---|---|---|---|---|---|
| | Blanc | 89576,000 | 0,000 | 0,00 | 100% |
| | T+ | 101450,333 | 11874,333 | 100,00 | 0% |
| | T- | 93017,667 | 3441,667 | 28,98 | 71% |
| **Extrait hydrolysé *peregrina*** | 2,0% | 98839,000 | 10687,333 | 90,00 | 10% |
| | 1,0% | 101400,667 | 17466,333 | 147,09 | NS |
| | 0,10% | 104515,333 | 24256,333 | 204,28 | NS |

Conclusion: L'extrait hydrolysé de Tourteau de *peregrina* n'est pas significativement impliqué dans l'inhibition directe de l'Angiotensine convertase 2 (ACE2). En considérant le tableau [32] de l'exemple 11 ci-dessous, il a été évalué l'amplitude d'action de cet extrait sur une autre convertase, à savoir la furine. L'action inhibitrice de l'extrait selon l'invention sur la furine est clairement démontrée et établie dans le tableau [32] de l'exemple 11. Cette inhibition spécifique démontrée sur une seule enzyme convertase renforce l'intérêt de l'hydrolysat de protéines selon l'invention en tant qu'inhibiteur spécifique de la furine convertase.

### Exemple 10 : Evaluation de l'effet anti-infectieux de l'extrait hydrolysé à inhiber in cellulo la furine en empêchant la pénétration de « Pseudo-virus à couronne spike SRAS-CoV-2 » : Modèle in cellulo sur cellules humaines HEK en présence de « Lentivirus pseudotypés SRAS-CoV-2 ».

La présente évaluation utilise deux composants principaux. D'une part, les cellules HEK293 stables clonales recombinantes expriment constitutivement ACE2 humain de pleine longueur (Genbank # NM_021804.3) avec une expression de surface de ACE2 confirmée par cytométrie en flux. D'autre part, les lentivirus pseudotypés SRAS-CoV-2 Spike ont été produits avec SRAS-CoV-2 Spike (numéro d'accès Genbank QHD43416.1) comme glycoprotéines d'enveloppe au lieu du VSV-G couramment utilisé. Ces pseudovirions contiennent également le gène de luciférase de luciole dirigé par un promoteur de CMV ; par conséquent, l'entrée de cellule médiée par le pic peut être mesurée de manière pratique via l'activité de rapporteur de luciférase. Le lentivirus pseudotypé SRAS-CoV-2 Spike peut être utilisé pour le dépistage des applications dans une installation de niveau de biosécurité 2.

Le matériel utilisé est le suivant :

**[Tableau 28]**

| Catalogue | Composants |
|---|---|
| 79951 | ACE2-HEK recomb cell line |
| 60187-1 | Milieu 1 de décongélaton |
| 79801 | Milieu de croissance 1N |
| 79942 | Spike (SARS-CoV-2) Pseudotyped Lentivirus (Luciferase Reporter) |
| 79943 | Bald Lentiviral Pseudovirion (Luciferase Reporter) |
| 60690-1 | ONE-Step luciferase assay system |
| AF933 | Humain/Souris/Rat/Hamster Anticorps ACE-2 (R&D Systems) |

### Étape 1: Mise en plaque de cellules ACE2-HEK

Les cellules ACE2-HEK sont décongelées dans le milieu de décongélation 1, amplifiées dans du milieu de croissance 1N, puis récoltées et mises dans des plaques de culture à 96 puits à fond plat blanc et transparent à 10.000 cellules / puits dans 50 µl de milieu de décongélation 1. Les cellules sont incubées pendant une nuit à 37 ° C.

### Étape 2: Test d'infection lentivirale pseudotypée

Le jour suivant, le contrôle visuel de l'homogénéité et de l'intégrité de la couche cellulaire est validé à l'aide d'un microscope inversé et les ingrédients de test suivants sont préparés selon les instructions suivantes :

**[Tableau 29]**

| | Nom de l'ingrédient /mode de co-incubation | Solubilité | Concentrations dans "puits" |
|---|---|---|---|
| Ingrédient 1 | Peregrina /ACE2-HEK | H20 / milieu de culture | 3% (C1), 0.5% (C2), 0.1% (C3) |

Dans une première étape, l'hydrolysat de protéine de *peregrina* est dilué à une concentration intermédiaire 11x dans le milieu de décongélation 1, pour le transfert ultérieur de 5 µl dans la plaque d'essai et la co-incubation avec des cellules ACE2-HEK. Après une période d'incubation de 30 min à 37 ° C, 5 µl de lentivirus pseudotypé non dilué (Bald ou S1-Spike) sont ajoutés aux puits d'analyse correspondants.

En tant que contrôle positif, le mAb bloquant ACE2 est utilisé à une concentration finale de 0,5 uM dans les puits d'analyse.

### Étape 3:

Après une période d'incubation de 48 heures, un volume de 50 µl de réactif luciférase est ajouté aux puits d'analyse et le signal luminescent est mesuré en utilisant le luminofluorimètre PolarStar Omega.

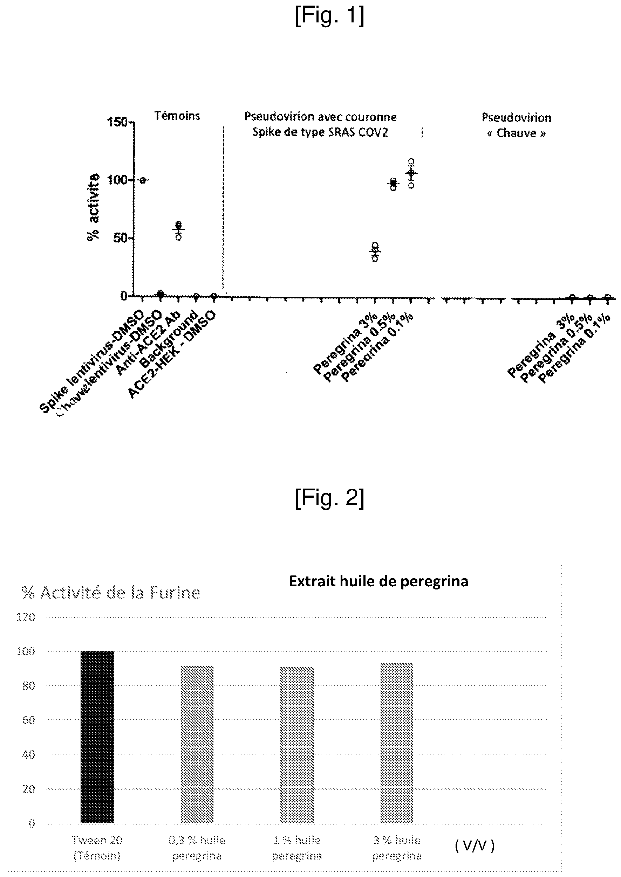

Les résultats sont donnés à la figure 1 pour l'effet anti-infectieux de l'hydrolysat de protéines de *peregrina* selon l'invention contre les pseudovirions du SRAS COV2.
Conclusion : L'hydrolysat de protéine de *peregrina* montre un effet anti-infectieux convaincant sur les pseudovirions du SRAS COV2 à partir d'une dose de 3% (C1) sur ce modèle d'étude. Il est démontré une inhibition marquée de 60% à la concentration C1 (3%).

Evaluation des bandes P1, P2, P3 identifiées à l'exemple 12 et du complexe P1+P2 sur la Furine convertase : Modèle de test acellulaire.

Les études conduites sur les bandes identifiées n'ont pas montré d'action inhibitrice sur la furine convertase sur toutes les concentrations testées avec P1, P2, P3 et P1+P2.

Conclusion : L'activité d'inhibition de la Furine Convertase n'est donc pas portée par l'une de ces bandes protéiques. Cela démontre que l'activité inhibitrice de la Furine Convertase est due à l'hydrolysat de protéines dans son totum obtenu par le procédé décrit.

L'hydrolysat de protéines dans son ensemble inhibe les protéines SPIKE de type SRAS-CoV2, notamment par l'inactivation de la furine convertase. Ces inhibitions apportent un caractère anti-infectieux et virostatique.

### Exemple 11 : Tests comparatifs de l'inhibition de la furine convertase par différentes préparations obtenues à partir des graines de Moringa peregrina

Le but de cette étude est d'évaluer l'effet inhibiteur de l'huile de *peregrina* obtenue par première pression à froid des graines avec coques, puis de l'extrait de *peregrina* par l'éthanol (96%) concentrant environ 1.1% de matière sèche, cette matière sèche étant elle-même constituée d'environ 55% en poids de 2,5-diformylfuran, 2.5% de furfural, 1.2% de myristate d'isopropyle, 4.7% d'acide palmitique, 11.1% d'acide oléique et 25.8% de triglycérides, et enfin de l'hydrolysat de protéines *peregrina* selon l'invention sur l'activité de la furine.

Protocole : L'extrait de *peregrina* à l'éthanol (96%) et l'hydrolysat de protéines *peregrina* ont été conservés à + 4 ° C, à l'abri de la lumière jusqu'à leur utilisation. L'huile de *peregrina* a été conservée à température ambiante dans une zone sombre.

Le produit de référence Le décanoyl-Arg-Val-Lys-Arg-CMK à 100 nm a été utilisé comme inhibiteur de référence de l'activité de la furine.

Le protocole d'incubation : La furine a été pré-incubée pendant 10 minutes à température ambiante en l'absence (Contrôle) ou en présence du produit de référence ou de concentrations croissantes des composés testés :
- Huile de *Peregrina* à 0.3, 1% et 3% (v / v).
- Extrait de *peregrina* à l'éthanol (96%) à 0.02%, 0,2% et 2% (v / v).
- Hydrolysat de protéines *peregrina* selon l'invention à 0.02%, 0.2% et 2% (v / v). A la fin de l'étape de pré-incubation, un substrat de furine a été ajouté et les conditions expérimentales ont été incubées à nouveau à température ambiante à l'abri de la lumière pendant 5 minutes. Toutes les expériences ont été réalisées en trois exemplaires.

Préparation des composés : L'extrait de *peregrina* à l'éthanol à 96% et l'hydrolysat de protéines de *peregrina* ont été solubilisés directement dans le tampon de dosage puis dilués afin d'atteindre la concentration à tester comme décrit ci-dessus. L'huile de *peregrina* a été solubilisée à 3% dans une solution de Tween20 à 0,05% dans du tampon de dosage. La solution a ensuite été diluée afin d'atteindre la concentration décrite ci-dessus.

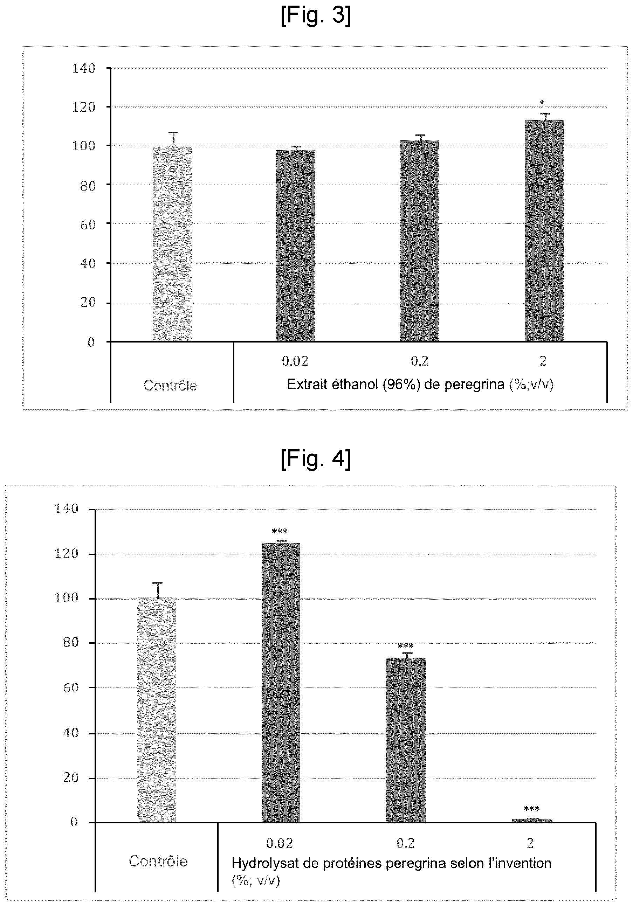

Protocole d'évaluation : Le clivage du substrat fluorescent de furine a été contrôlé pendant 5 minutes après l'addition du substrat en lisant la fluorescence à 485 nm / 535 nm. g. Statistiques : Les résultats sont exprimés en RFU (Relative Fluorescent Units) +/- S.D. (Écart-type). La signification statistique de la différence observée entre les groupes « Contrôle » et « Produit de référence » a été évaluée par un test de Student (p <0,001). La signification statistique de la différence observée entre les groupes « Contrôle » et « Composé test » a été évaluée par une analyse ANOVA unidirectionnelle, suivie d'un test HolmSidak ( p <0,05). Les résultats pour l'huile *peregrina* sont donnés à la figure 2, ceux de l'extrait éthanol 96° *peregrina* à la figure 3 et enfin ceux de l'hydrolysat de protéines *peregrina* selon l'invention à la figure 4.

### Résultats :

**[Tableau 30]**

| Concentration huile de *peregrina* | Activité de la furine convertase |
|---|---|
| 0.3% (v :v) | 91.2%** |
| 1% (v :v) | 90.6%** |
| 3% (v :v) | 92.8%** |

**[Tableau 31]**

| Concentration extrait éthanol 96° *peregrina* | Activité de la furine convertase |
|---|---|
| 0.02% (v :v) | 97.3%* |
| 0.2% (v :v) | 102.0%* |
| 2.0% (v :v) | 112.8%* |

**[Tableau 32]**

| Concentration hydrolysat de protéines *peregrina* | Activité de la furine convertase |
|---|---|
| 0.02% (v :v) | 124.9%*** |
| 0.2% (v :v) | 73.2%*** |
| 2.0% (v :v) | 1.2%*** |

| | |
|---|---|
| *Significativité statistique (p>0.05) **: Significativité statistique (p<0.01) ***: Significativité statistique (p<0.001) | |

Conclusion : La présente étude nous a permis de montrer que seul l'hydrolysat de protéines *peregrina* selon l'invention peut inhiber significativement l'activité de la furine de 98,8% à une concentration de 2%.

### Exemple 12 : Chromatographie séparative sur gel électrophorèse de l'hydrolysat de protéines de peregrina - Bandes protéiques.

L'électrophorèse en gel de polyacrylamide contenant du dodécysulfate de sodium est appelée électrophorèse SDS-PAGE. II s'agit d'une technique consistant à faire migrer des protéines dénaturées par saturation de charge négative via le SDS dans un gel de polyacrylamide, sous l'influence d'un champ électrique, permettant ainsi leur séparation. C'est une technique dénaturante qui dissocie les complexes protéiques non-covalents grâce à l'emploi d'un détergent ionique chargé négativement (le SDS). Ce détergent se lie indifféremment par liaisons hydrophobes à deux acides aminés. Cette technique permet ainsi d'analyser les protéines et de les séparer en fonction de leur masse moléculaire.

### Chromatographie séparative par méthode électrophorèse :

### Dépôts :

- MW = marqueur de taille
- puits 1 : Extrait hydrolysé de tourteau de *Peregrina*
- puits 2 : surnageant de l'Extrait hydrolysé de tourteau de *Peregrina*
- puits 3 : culot de l'Extrait hydrolysé de tourteau de *Peregrina*

L'extrait hydrolysé dans le puit 1 montre bien toutes les bandes protéiques attendues.

Le puits 2 (surnageant) présente des bandes atténuées notamment sur les poids moléculaires intermédiaires et les plus élevés ; le puits 2 semble avoir concentré les poids moléculaires les plus bas apparentés notamment aux volatils.

Le puits 3 (culot) présente clairement les bandes des poids moléculaires les plus élevés (> à 75 000 Da), puis une bande appelée P3 d'environ 23 000 Da, puis une bande appelée P2 comprise entre 10 000 et 17 000 Da et enfin une bande inférieure à 10 000 Da appelée P1, estimée entre 4 000 et 6 000 Da.

### Préparation Rotavapor :

- Les volatils sont logiquement dans le surnageant avec les composés les plus « légers » ; 10 ml de surnageant sont récupérés pour être additionnés de 10 ml d'éthanol 96° ;
- Le mélange est extrait sous vide à 45°C ; les composés volatils sont condensés dans un ballon sous vide.
- Le condensat concentré en volatils sera passé en GC/FID après micro-extraction SPME

Etude en Chromatographie Gazeuse après une méthode d'extraction SPME en détection FID
Aucun composé volatil n'a été détecté.

Conclusion : L'isolement des bandes protéiques révèle que les composés volatils ne sont pas liés aux protéines ; les composés volatils ne participent pas au cortège moléculaire des bandes protéiques les plus petites.

Détermination de trois bandes protéiques : Les bandes P1 (moins de 10 000 Da) et P2 (entre 10 000 et 17 000 Da) et P3 (environ 23 000 Da) sont préparées et passées en analyse chromatographique en phase liquide couplée à la spectrométrie de masse (LC MS/MS).

Les résultats sont obtenus à partir de 2 logiciels bio-informatiques spécialisés dans la reconnaissance protéique chez les plantes (Mascot et Peaks):
Les résultats d'identification ne permettent pas d'identifier précisément ces protéines ; nous sommes en présence de protéines actuellement non décrites dans les bases de données sus-mentionnées.

### Exemple 13 : Formulation d'un produit dermatologique anti-fibrotique (nettoyant liquide)

**[Tableau 31]**

| **Ingrédients** | **%** |
|---|---|
| Eau | 80,7000 |
| Sodium coco-sulfate | 5,0000 |
| Sodium cocoyl isethionate | 4,0000 |
| Bentonite | 3,7800 |
| Caprylic / Capric triglyceride | 2,0000 |
| Hydrolysat de protéines *peregrina* selon l'invention | 2,0000 |
| Gluconolactone | 0,7500 |
| Sodium benzoate | 0,5450 |
| Parfum | 0,5000 |
| Gomme de Xanthane | 0,2700 |
| Sodium stearoyl glutamate | 0,2250 |
| Acide Citrique | 0,2250 |
| Calcium gluconate | 0,0050 |

### Exemple 14: Formulation d'un produit dermatologique traitant la fibrose topique (produit de soin non rincé)

**[Tableau 32]**

| **Ingrédient** | **%** |
|---|---|
| Eau | 71,5000 |
| Caprylic / Capric triglyceride | 18,0000 |
| Bentonite | 4,2000 |
| Cetearyl alcohol | 1,5000 |
| Hydrolysat de protéines *peregrina* selon invention | 2,0000 |
| Gluconolactone | 0,7500 |
| Sodium benzoate | 0,5450 |
| Gomme Xanthane | 0,5000 |
| Parfum | 0,5000 |
| Sodium stearoyl glutamate | 0,2500 |
| Acide Citrique | 0,2500 |
| Calcium gluconate | 0,0050 |

### Exemple 15 : Formulation d'une composition injectable par voie sous-cutanée

Formulation d'un produit pour administration sous-cutanée : extrait sec selon l'invention (contenant 60% d'hydrolysat de protéine sur un support d'inuline) conditionné en flacon mono-dose sous gaz inerte prêt à être solubilisé par un milieu physiologique.

### Exemple 16 : Formulation d'une composition injectable par voie sous-cutanée

Formulation d'un produit injectable liquide : extrait liquide selon l'invention dosé à 5% dans un milieu physiologique conditionné en condition stérilisante notamment par filtration sous vide avec un seuil de coupure à 0.45µm.

Exemple 17 : Formulation sous forme de patch (Pansement ou dispositif médical externe collé à la peau, imbibé du principe actif qu'il libère lentement, permettant un effet de diffusion lente du principe actif).

### Exemple 18 : Formulation d'un médicament en gélule

Médicament antifibrotique en gélule de 750 mg contenant 100 mg de pipérine, 300 mg d'extrait sec selon l'invention (contenant 60% d'hydrolysat de protéines sur un support d'inuline), 100 mg d'acide boswellique, 250 mg de carbonate de calcium.)

### Exemple 19 : Formulation d'un médicament en comprimé

Médicament antifibrotique en comprimé de 1 g : 300 mg d'extrait sec selon l'invention (contenant 60% d'hydrolysat de protéines sur un support d'inuline), 400 mg de carbonate de calcium contenant 200 UI de vitamine D, 150 mg de gluconate de magnésium, 80 mg d'inuline et 70 mg de stéarate de magnésium.

Exemple 20 : Formulation d'un médicament en spray nasal (solution contenant le principe actif dans un dispositif médical propulsant par brumisation la solution active dans la cavité nasale).

Exemple 21 : Tests toxicologiques de l'hydrolysat de protéines selon l'invention Préparation de l'hydrolysat de protéines conformément à l'exemple 1 : Des graines non décortiquées de *Moringa peregrina* (Forssk.) Fiori récoltées à maturité du fruit ont été séchées pour obtenir un taux d'humidité interne inférieur à 8% et préférentiellement autour de 6%, puis pressées avec une presse mécanique à vis sans fin, de sorte à séparer l'huile du reste de la graine pour obtenir d'une part l'huile vierge et d'autre part un tourteau. Le tourteau est alors isolé sous forme de boudins prédécoupés en morceau de 1 à 2 cm. En suivant le protocole décrit à l'exemple 1, on obtient l'extrait liquide que l'on utilise non dilué dans les tests qui suivent.

### 1. Détermination de l'activité mutagène sur la souche bactérienne Salmonella typhimurium (TA 100) - Test de mutation bactérienne inverse

Le test s'est déroulé en 3 phases principales :
- Une expérience préliminaire est réalisée afin d'évaluer la cytotoxicité de l'élément à tester et de sélectionner la gamme de doses pour les expériences ultérieures,
- Une première expérience de génotoxicité (Test 1), avec et sans activation métabolique, avec incorporation directe du système de test et du test (ou des contrôles) sur gélose minimale, sur la plage de doses définie par l'étude préliminaire,
- Une deuxième expérience (Test 2), avec pré-incubation du système de test et de l'élément de test (ou des contrôles), avec et sans activation métabolique, avec des niveaux de dose définis par le responsable de l'étude après analyse des résultats de la première expérience. Cette seconde expérience a été réalisée afin de confirmer ou de compléter les résultats de la première, en particulier lorsque des résultats équivoques ou négatifs ont été obtenus.
Les dilutions de l'extrait selon l'exemple 1 ont été préparées dans l'eau pour réaliser le test de cytotoxicité.

Ce dernier a été réalisé sur la souche *Salmonella typhimurium* TA100 aux concentrations de 5000, 1600, 500, 160 et 50 µg / plaque, avec et sans S9-Mix.
Les réactifs utilisés pour la préparation de S9-Mix ont été préparés selon les instructions suivantes :

**[Tableau 33]**

| | Concentration finale |
|---|---|
| MgCl2 (0.4 M) + KCI (1.65 M) | 8 mM + 33 mM |
| Glucose 6 Phosphate (0.2 M) | 5 mM |
| NADP (0.1 M) | 4 mM |
| Tampon Phosphate pour S9-Mix (pH 7.4 - 0.2 M) | 0.1 M |
| S9 fraction | 10% |
| Eau | Ajuster pour concentration finale |

Les bactéries ont été exposées à l'extrait de test selon l'invention avec et sans système d'activation métabolique. Le système métabolique utilisé est une fraction post-mitochondriale améliorée par cofacteur (S9). Cette fraction S9, une fraction microsomale d'homogénat de foie de rat Sprague Dawley traité avec un inducteur enzymatique, est préparée selon MARON, D.M. et al., 1983, Revised Methods for the Salmonella Mutagenicity Test, Mutation Research/Environmental Mutagenesis and Related Subjects, 113, p .173-215), et a été fournie par MOLTOX TM. Elle est conservée à une température inférieure à -70°C. La fraction microsomale S9 a été utilisée à la concentration de 10% dans S9-Mix. Le protocole appliqué a été le suivant :
- Dans 3 tubes d'hémolyse, a été introduit :
   ∘ dosage sans activation métabolique:
      - 0,1 ml des différentes concentrations des éléments de test,
      - 0,5 ml de tampon phosphate stérile 0,2 M, pH 7,4,
      - 2 ml de top agar pour S. *typhimurium,*
      - 0,1 ml d'inoculum bactérien (TA100).
   ∘ dosage avec activation métabolique:
      - 0,1 ml des différentes concentrations des éléments de test,
      - 2 ml de top agar pour S. *typhimurium,*
      - 0,1 ml d'inoculum bactérien (TA100),
      - 0,5 ml de S9-Mix.
- Mélanger et verser sur la surface de la gélose de fond préalablement répartie dans des boîtes de Pétri.
- Incuber à 37 ° C ± 2 ° C pendant 48 à 72 heures.
   Ces dosages ont été réalisés pour chaque test : test préliminaire de cytotoxicité, test 1 et test 2. Le contrôle non traité, les contrôles négatifs et les contrôles positifs réalisés au cours de la méthode de pré-incubation ont été incubés pendant 20 à 30 minutes à 37 ° C ± 2 ° C avant de verser la gélose supérieure.
   Le protocole appliqué a été le suivant :
- Dans 4 fractions de 2 ml d'agar top pour S. *typhimurium,* introduire :
   ∘ 0,1 ml de tampon phosphate 0,2 M, pH 7,4,
   ∘ 0,1 ml de solvant,
   ∘ 0,1 ml de S9-Mix.
   ∘ 0,1 ml de la préparation de l'élément à tester à la concentration la plus élevée,
- Une fraction de 2 ml d'agar top pour *S*. *typhimurium* est utilisée pour contrôler sa stérilité.
- Mélanger et verser sur la surface de la gélose de fond préalablement répartie dans des boîtes de Pétri.
- Incuber à 37 ° C ± 2 ° C pendant 48 à 72 heures.
- Le test est réalisé en triple.
- Aucune croissance bactérienne ne doit être observée.
Pour au moins 5 concentrations de l'extrait à tester, un test sans activation métabolique et un test avec activation métabolique ont été réalisés.

### Expression des résultats et interprétation

De nombreux critères permettent de déterminer si un résultat est positif, notamment une augmentation du nombre de révertants corrélée à la dose d'item à tester, ou une augmentation reproductible du nombre de révertants à une ou plusieurs concentrations, avec ou sans activation métabolique.
- L'élément à tester est considéré comme mutagène si à l'issue des étapes de vérification, il a été obtenu, de manière reproductible, une relation dose-effet sur une ou certaines des 5 souches avec et / ou sans activation métabolique. La mutagénicité n'est prise en compte pour une concentration donnée que lorsque le nombre de révertants est au moins égal au double du taux de réversion spontanée pour les souches TA98, TA100 et TA102 (R ≥ 2) et au triple du taux spontané de réversion pour les souches TA1535 et TA1537 (R ≥ 3).
- L'élément de test est considéré comme non mutagène si, à l'issue du test 1 et du test 2, le taux de révertants est toujours resté inférieur au double du taux de réversion spontanée pour toutes les concentrations de l'élément de test testé, pour les souches TA98, TA100 et TA102 (R <2) et inférieur au triple du taux de réversion spontanée pour les souches TA1535 et TA1537 (R <3), avec et sans activation métabolique, et à condition qu'il ait été vérifié que l'absence de l'effet mutagène n'est pas liée à la toxicité des concentrations testées.
L'étude préliminaire n'a montré aucune cytotoxicité de l'élément de test ; par conséquent, cette gamme de concentrations a été utilisée pour le test de génotoxicité 1.

En fonction du résultat obtenu pour le test 1, il a été décidé d'utiliser la même gamme de dilution pour le test 2. L'analyse des révertants montre que :
- Aucun effet cytotoxique n'a été observé,
- Aucune concentration de l'extrait à tester n'a montré un rapport R supérieur ou égal au moins au double du taux de réversion spontanée pour TA98, TA100 et TA102 et au triple du taux de réversion spontanée pour TA1535 et TA1537, avec et sans activation métabolique,
- Aucune réponse à la dose n'a été observée, quels que soient le système de test ou les conditions du test.

Au vu des résultats obtenus au cours de cette étude, l'hydrolysat de protéines selon l'exemple 1 peut être considéré comme ne présentant aucune activité mutagène ni pro-mutagène.

### 2. Test de photo-toxicité in vitro 3T3 NRU

Le principe du test a pour base la comparaison de la cytotoxicité de l'hydrolysat de protéines selon l'exemple 1 en présence et en absence d'une dose non cytotoxique d'UVA, sur des cellules en culture. La cytotoxicité est évaluée par la détermination de la viabilité cellulaire à l'aide d'un colorant vital : le rouge neutre, 24h après le traitement avec les éléments de référence et l'hydrolysat de protéines *peregrina* selon l'invention avec ou sans irradiation aux UVA. Les cellules utilisées sont des fibroblastes d'embryon de souris de lignée Balb/c 3T3 clone 31 (ATCC - CCL163). Le contrôle positif est une solution de chlorpromazine (Numéro CAS : 69-09-0). Le contrôle négatif est un diluant de l'extrait testé et de référence (solution saline tamponnée +/- 1% solvant). L'hydrolysat de protéines *peregrina* a été testé à 8 concentrations sur au moins quatre puits de culture par concentration étudiée, en présence ou en absence d'UVA. Les fibroblastes ont été trypsinés et deux plaques de culture 96 puits ont été ensemencées à raison de 100 µl d'une suspension cellulaire à 2.10⁵ cellules/ml (soit 2.10⁶cellules par puits) dans du milieu de culture complet.

Les plaques ensemencées ont été incubées à l'étuve 24 heures à 37°C, 5% CO₂. A la fin de l'incubation, la semi-confluence du tapis cellulaire a été vérifiée. Les dilutions ont été préparées juste avant leur dépôt sur les cellules. Le PH de la plus forte concentration a été mesuré ; il est compris entre 6,5 et 7,8. Le milieu de culture a été éliminé ; chaque puits a été préalablement rincé délicatement avec 150 µl de PBS maintenu à température ambiante avant d'être traité avec 100 µl de chaque dilution de l'extrait ou de référence. Les plaques de cultures ont été incubées à l'obscurité durant 1h ± 5 minutes à 37°C et 5 % de CO₂. L'irradiation a été réalisée à l'aide d'un irradiateur solaire BIO SUN (Vilber Lourmat RMX3W). Le BIO SUN est un système qui contrôle l'irradiation UV à l'aide d'un microprocesseur programmable. Le système suit en continu l'émission de lumière UV. L'irradiation s'arrête automatiquement quand l'énergie délivrée est égale à l'énergie programmée. L'irradiation spectrale du dispositif à l'essai a été mesurée dans la gamme de longueurs d'onde 250 à 700 nanomètres avec un spectroradiomètre calibré.

Une des 2 plaques a été irradiée avec son couvercle à température ambiante, et l'autre plaque a été protégée des UVA et conservée à température ambiante le temps de l'irradiation. Après l'irradiation, le milieu de traitement a été aspiré et les cellules ont été rincées. Puis 100 µl de milieu de culture complet ont été ajoutés doucement et les plaques ont été incubées pendant 18 à 22h à 37°C et 5 % de CO₂. Le lendemain la viabilité cellulaire (croissance, morphologie, intégrité de la monocouche) a été évaluée par observations sur un microscope à contraste de phase. Le milieu de culture a été éliminé, chaque puits a été préalablement rincé et maintenu à température ambiante avant d'être traité avec 100 µl de la solution de coloration. Les plaques ont été remises à l'incubateur durant 3h dans les mêmes conditions. La solution colorante a été éliminée et les cellules ont été rincées, puis la solution de rinçage a été enlevée et 150 µl de solution de désorption ont été ajoutés dans chaque puits. Les plaques ont été agitées jusqu'à la solubilisation complète des cristaux. Les absorbances ont été mesurées à 450 nm.

### Validation du test :

La sensibilité des cellules aux UVA est contrôlée tous les 10 passages environ, par évaluation de leur viabilité après exposition à des doses croissantes d'irradiation. Les cellules sont mises en culture à la densité utilisée dans l'essai. Elles sont irradiées le jour suivant à la dose de 2,5 et 9 J/cm² et la viabilité cellulaire est déterminée un jour plus tard à l'aide du test NRU. Les cellules répondent aux critères de qualité si leur viabilité après irradiation à 5 J/cm² UVA est supérieure ou égale à 80 % de la viabilité des témoins maintenus à l'obscurité ; à la dose la plus forte de 9 J/cm² UVA, la viabilité doit être au moins égal à 50% de celle des témoins maintenus à l'obscurité.

### Résultats :

Le contrôle négatif a une absorbance supérieure ou égale à 0,4. La chlorpromazine, contrôle positif, présente une Cl₅₀ comprise entre 0,1 et 2 µg/ml en présence d'UVA et comprise entre 7 et 90 µg/ml en absence d'UVA. Ces résultats permettent de valider le test. La concentration de l'hydrolysat de protéines *peregrina* selon l'exemple 1 donnant 50% de mort cellulaire en présence ou en absence d'UVA ne peut être estimée. La mortalité n'a jamais atteint 50%. La concentration de l'hydrolysat de protéines *peregrina* donnant 50% de viabilité cellulaire en présence ou en absence d'UVA ne peut être estimée. La viabilité est toujours supérieure à 50%.

Conclusion : Dans les conditions expérimentales retenues l'hydrolysat de protéines *peregrina* selon l'invention peut être considéré comme non phototoxique.

### 3. Evaluation du potentiel irritant oculaire par l'étude de la cytotoxicité in vitro selon la méthode de relargage du rouge neutre sur lignée cellulaire SIRC

Cette étude in vitro a pour base l'évaluation de la cytotoxicité de l'hydrolysat de protéines *peregrina* par détermination de la concentration entrainant 50% de mortalité cellulaire (Cl₅₀) sur une monocouche cellulaire à l'aide de la technique de relargage du rouge neutre. Les cellules utilisées sont des fibroblastes de cornée de lapin SIRC (ATCC - CCL60) exemptes de mycoplasmes.

L'hydrolysat de protéines *peregrina* a été dilué dans du sérum physiologique à 25% et 50%. Les fibroblastes ont été trypsinés et deux plaques de culture 24 puits ont été ensemencées à raison de 1 ml d'une suspension cellulaire à 2.10⁵ cellules/ml dans du milieu de culture complet. Les plaques ensemencées ont été incubées à l'étuve une nuit à 37°C et 5% CO₂. A la fin de l'incubation, la confluence du tapis cellulaire a été vérifiée. La solution de coloration a été préparée à 0,5 mg/ml dans du milieu de culture complet. Le milieu de culture a été éliminé ; 1 ml de la solution colorante a été déposée dans chaque puits. Les plaques ont été remises à l'incubateur à 37°C et 5% CO2 pendant 3h +/- 15 minutes. Après ce temps de contact, la solution colorante a été éliminée et remplacée par 1 ml de milieu de culture complet par puits. Les plaques ont été maintenues à température ambiante pendant au moins 30 minutes afin de stabiliser le système avant le contact avec l'extrait ou la référence. Chaque puits a été rincé avec 2 ml de PBS, maintenu à température ambiante, puis 500 µl de chaque dilution d'hydrolysat de protéines *peregrina* ou de référence ont été déposées au contact du tapis cellulaire. Le temps de contact a été de 60 secondes (30 secondes pour le contrôle positif). Le traitement a été réalisé puits par puits avec déclenchement du chronomètre au moment du dépôt de l'hydrolysat de protéines *peregrina* ou la référence. La plaque a été agitée manuellement pendant toute la durée du traitement. Après 55 secondes (ou 25 secondes pour le contrôle positif), la dilution a été aspirée. A 60 ou 30 secondes précises, 5 rinçages successifs ont été effectués (5 x 2 ml de PBS maintenus à température ambiante). Le surnageant a été aspiré après chaque rinçage et après le dernier rinçage les puits sont restés dépourvus de milieu en attendant la phase de révélation. Après traitement complet de la plaque de culture, 1 ml de la solution de désorption a été déposée dans chaque puits. La plaque est agitée pendant environ 15 minutes jusqu'à l'obtention d'une coloration homogène. Les solutions obtenues pour chaque puits de culture ont été prélevées et réparties dans 2 puits d'une plaque 96 puits, soit 150 µl/puits.

### Résultats :

La concentration de l'hydrolysat de protéines *peregrina* donnant 50% de mort cellulaire a été évaluée à >50%. Le pourcentage de mort cellulaire à 50% d'hydrolysat de protéines *peregrina* a été évalué à 17%.

Conclusion : Dans les conditions expérimentales retenues, la cytotoxicité de l'hydrolysat de protéines *peregrina* selon l'invention peut être classée comme cytotoxicité négligeable.

### 4. Evaluation de la compatibilité cutanée d'un hydrolysat de protéines peregrina selon l'invention après application unique sous pansement occlusif pendant 48 heures sous contrôle dermatologique.

Le but de cette étude est d'évaluer le degré de compatibilité cutanée de l'hydrolysat de protéines *peregrina* par test épicutané, réalisé au niveau de la face antéro-externe du bras pendant 48 heures ; et d'une manière générale évaluer la capacité de l'hydrolysat de protéines à maintenir la peau en bon état. 10 volontaires sains, féminin ou masculin, âgés de 18 à 65 ans, n'ayant ni la peau sèche ni la peau sensible et indemnes de toute lésion dermatologique sur la zone de traitement devaient être inclus dans l'étude. La compatibilité cutanée de l'hydrolysat de protéines *peregrina,* préparé sous forme de lotion avec 5% de l'hydrolysat de protéines *peregrina* selon l'exemple 1 et 95% d'un mélange Propanediol/Sorbitol, a été évaluée 48 heures après l'application initiale entre 30 à 40 minutes après le retrait du pansement. Le comptage des réactions cutanées (érythème et œdème) a été effectué de 0 à 3 selon les échelles suivantes :

**[Tableau 34]**

| Cotation | Erythème (E) | Œdème (Oe) |
|---|---|---|
| 0 | Aucun érythème | Aucun œdème |
| 0,5 | Érythème à peine perceptible, coloration très légèrement rosée sur une partie de la zone depatchage | Œdème palpable à peine perceptible |
| 1 | Érythème léger, coloration rosée sur toute la surface de patchage | Œdème palpable et visible |
| 2 | Érythème modéré, coloration nette sur toute la surface de patchage | Œdème net avec ou sans papules ou vésicules |
| 3 | Érythème important, coloration intense sur toute la zone de patchage | Œdème important diffusant en dehors de la zone de patchage avec ou sans papules ou vésicules |

Toute autre réaction cutanée (bulles, papules, vésicules, sécheresse, desquamation, rugosité, effet savon...) a été évaluée selon l'échelle suivante et rapportée de manière descriptive :
- 0 : aucune réaction,
- 0,5 : très léger
- 1: léger
- 2 : modéré
- 3 : important.

A la fin de l'étude, un indice d'irritation moyen (I.I.M.) a été calculé selon la formule suivante : I.I.M. = Somme des réactions cutanées (E + Oe+ bulles + papules + vésicules) / Nombre de volontaires analysés

L'I.I.M. obtenu a permis de classer l'hydrolysat de protéines *peregrina* testé selon le barème présenté ci-après :

| | |
|---|---|
| I.I.M. ≤ 0,20 | Non irritant |
| 0,20 < I.I.M. ≤ 0,50 | Légèrement irritant |
| 0,50 < I.I.M. ≤ 2 | Moyennement irritant |
| 2 < I.I.M. ≤ 3 | Très irritant |

Résultats : L'Indice d'Irritation Moyen (I.I.M.) de l'hydrolysat de protéines *peregrina* est égal à : 0.

Conclusion : L'hydrolysat de protéines *peregrina* peut être considéré comme non irritant après 48 heures consécutives d'application chez 12 volontaires.

### Conclusion générale des tests :

Les résultats des tests effectués ci-dessus sont concluants et démontrent pour l'hydrolysat de protéines *peregrina* selon l'exemple 1 :
1) Les tests d'irritation oculaire et cutanée sont négatifs
2) Les tests de phototoxicité sont négatifs
3) Les tests de mutagénicité sont négatifs.

La sécurité de l'hydrolysat de protéines *peregrina* selon l'invention est démontrée et idéale pour une utilisation dermatologique à grande échelle sans restriction sur les populations cibles.

## Revendications

1. Procédé d'obtention d'un hydrolysat de protéines du tourteau des graines de *Moringa peregrina,* **caractérisé en ce qu'**il comprend les étapes suivantes selon lesquelles :
a) on recueille les graines non décortiquées à maturité du fruit de *Moringa peregrina* que l'on sèche pour obtenir un taux d'humidité interne inférieur à 8%,
b) on presse les graines séchées de sorte à séparer l'huile du reste de la graine de sorte à obtenir le tourteau comprenant moins de 6% en poids d'huile résiduelle,
c) on broie le tourteau obtenu à l'étape b),
d) on met en dispersion en phase aqueuse le tourteau broyé obtenu à l'étape
c) dans une proportion respective de 90,9/9,1 (masse/masse),
e) on réalise une protéolyse chimique de la dispersion aqueuse obtenue à l'étape d) pendant une durée d'environ 2 heures, à pH supérieur à 13 et à une température comprise entre 16 et 25°C,
f) on neutralise la protéolyse pour stabiliser l'hydrolysat de protéines obtenu,
g) on récupère l'hydrolysat de protéines par séparation solide/liquide,
h) on purifie l'hydrolysat de protéines par ultrafiltration et/ou nanofiltration effectuée avec un seuil de coupure compris entre 100 et 25000 Da.

2. Procédé selon la revendication 1, **caractérisé en ce que** la nanofiltration est effectuée de manière à séparer 3 bandes de l'hydrolysat de protéines comprenant une bande P1 dont le poids moléculaire est inférieur à 10 000 Da, une bande P2 dont le poids moléculaire est compris entre 10 000 et 17 000 Da et une bande P3 dont le poids moléculaire est d'environ 23 000 Da.

3. Procédé selon l'une des revendications 1 et 2, **caractérisé en ce que** l'étape h) de nanofiltration est effectuée avec un seuil de coupure compris entre 1 500 Da et 5 000 Da, de préférence avec un seuil de coupure compris entre 3 000 Da et 4 500 Da.

4. Procédé selon l'une des revendications 1 et 2, **caractérisé en ce que** l'étape h) de nanofiltration est effectuée avec un seuil de coupure compris entre 10 000 Da et 17 000 Da.

5. Procédé selon l'une des revendications 1 et 2, **caractérisé en ce que** l'étape h) de nanofiltration est effectuée avec un seuil de coupure compris entre 17 000 Da et 25 000 Da.

6. Procédé selon l'une des revendications 1 à 5, **caractérisé en ce que** i) on effectue une lyophilisation de l'hydrolysat de protéines obtenu à l'étape h).

7. Hydrolysat de protéines du tourteau des graines non décortiquées et récoltées à maturité du fruit de *Moringa peregrina,* **caractérisé en ce qu'**il comprend une fraction P1 majoritaire (masse/masse) de dérivés d'acides aminés, d'acides aminés, de peptides et glycopeptides dont le poids moléculaire est compris entre 1 500 Da et 5 000 Da, une fraction P2 d'environ 20% (masse/masse) dont le poids moléculaire est compris entre 10 000 à 17 000 Da et une fraction P3 d'environ 20% (masse/masse) dont le poids moléculaire est d'environ 23 000 Da, **en ce qu'**il est obtenu par protéolyse chimique à pH supérieur à 13 pendant une durée d'environ 2 heures à une température comprise entre 16 et 25°C et **en ce qu'**il est liquide et présente une densité supérieure à 1.

8. Hydrolysat de protéines du tourteau des graines non décortiquées et récoltées à maturité du fruit de *Moringa peregrina,* **caractérisé en ce qu'**il comprend la fraction P1 majoritaire (masse/masse) de dérivés d'acides aminés, d'acides aminés, de peptides et glycopeptides dont le poids moléculaire est compris entre 1 500 Da et 5 000 Da, **en ce qu'**il est obtenu par protéolyse chimique à pH supérieur à 13 pendant une durée d'environ 2 heures à une température comprise entre 16 et 25°C suivi d'une nanofiltration effectuée avec un seuil de coupure compris entre 1 500 Da et 5 000 Da et **en ce qu'**il est liquide et présente une densité supérieure à 1.

9. Hydrolysat de protéines du tourteau des graines non décortiquées et récoltées à maturité du fruit de *Moringa peregrina,* **caractérisé en ce qu'**il comprend la fraction P2 d'environ 20% (masse/masse) de dérivés d'acides aminés, d'acides aminés, de peptides et glycopeptides dont le poids moléculaire est compris entre 10 000 Da et 17 000 Da, **en ce qu'**il est obtenu par protéolyse chimique à pH supérieur à 13 pendant une durée d'environ 2 heures à une température comprise entre 16 et 25°C suivi d'une nanofiltration effectuée avec un seuil de coupure compris entre 10 000 Da et 17 000 Da et **en ce qu'**il est liquide et présente une densité supérieure à 1.

10. Hydrolysat de protéines du tourteau des granes non décortiquées et récoltées à maturité du fruit de *Moringa peregrina,* **caractérisé en ce qu'**il comprend la fraction P3 d'environ 20% (masse/masse) de dérivés d'acides aminés, d'acides aminés, de peptides et glycopeptides dont le poids moléculaire est d'environ 23 000 Da, **en ce qu'**il est obtenu par protéolyse chimique à pH supérieur à 13 pendant une durée d'environ 2 heures à une température comprise entre 16 et 25°C suivi d'une nanofiltration effectuée avec un seuil de coupure compris entre 17 000 Da et 25 000 Da et **en ce qu'**il est liquide et présente une densité supérieure à 1.

11. Hydrolysat de protéines du tourteau des graines non décortiquées et récoltées à maturité du fruit de *Moringa peregrina,* **caractérisé en ce qu'**il comprend en combinaison l'hydrolysat de protéines selon la revendication 8 et l'hydrolysat de protéines selon la revendication 9.

12. Hydrolysat de protéines du tourteau des graines de *Moringa peregrina* selon l'une des revendications 7 à 11 pour son application comme médicament.

13. Composition pharmaceutique ou dermatologique, **caractérisée en ce qu'**elle comprend à titre d'agent actif, une quantité efficace d'un hydrolysat de protéines du tourteau des graines de *Moringa peregrina* selon l'une des revendications 7 à 11, et un excipient physiologiquement acceptable.

14. Composition pharmaceutique selon la revendication 13, **caractérisée en ce qu'**elle est formulée pour être ingérée.

15. Composition dermatologique selon la revendication 13, **caractérisée en ce qu'**elle est formulée pour être appliquée par voie locale sur la peau et les muqueuses.

16. Composition selon la revendication 13 à 15, **caractérisée en ce que** l'hydrolysat de protéines de tourteau des graines de *Moringa peregrina* est présent dans la composition à une concentration de 0.0001 à 40% en poids par rapport au poids total de la composition.

17. Composition selon l'une des revendications 13 à 16 pour son utilisation en tant que médicament pour le traitement de maladies fibrotiques et le traitement de l'inflammation, **caractérisée en ce qu'**elle comprend comme agent actif une quantité efficace de l'hydrolysat de protéines selon la revendication 8 ou 9, de préférence selon la revendication 9.

18. Composition selon l'une des revendications 13 à 16 pour son utilisation en tant que médicament pour le traitement du cancer, **caractérisée en ce qu'**elle comprend comme agent actif une quantité efficace de l'hydrolysat de protéines selon la revendication 10.

19. Composition selon l'une des revendications 13 à 16 pour son utilisation en tant que médicament pour le traitement de maladies infectieuses de type virales pour inhiber les protéines Spike-COV2 du SRAS-COV2, **caractérisée en ce qu'**elle comprend comme agent actif une quantité efficace de l'hydrolysat de protéines selon la revendication 7.

20. Composition selon l'une des revendications 13 à 16 pour son utilisation en tant que médicament pour le traitement de la fibrose de la peau et du lentigo, **caractérisée en ce qu'**elle comprend comme agent actif une quantité efficace de l'hydrolysat de protéines selon la revendication 11.

## Patentansprüche

1. Verfahren zur Herstellung eines Proteinhydrolysats aus dem Presskuchen der Samen von *Moringa peregrina,* **dadurch gekennzeichnet, dass** dies die folgenden Schritte umfasst, gemäß welchen:
a) die ungeschälten, reifen Samen der Frucht von *Moringa peregrina* gesammelt und getrocknet werden, um einen inneren Feuchtigkeitsgehalt von weniger als 8 % zu erhalten,
b) die getrockneten Samen so gepresst werden, dass das Öl vom Rest der Samen getrennt wird, um den Presskuchen zu erhalten, der weniger als 6 Gew.-% Restöl umfasst,
c) der in Schritt b) erhaltene Presskuchen zerquetscht wird,
d) der in Schritt c) erhaltene gemahlene Presskuchen in wässriger Phase in einem Verhältnis von 90,9/9,1 (Masse/Masse) dispergiert wird,
e) eine chemische Proteolyse der in Schritt d) erhaltenen wässrigen Dispersion während einer Dauer von etwa 2 Stunden bei einem pH-Wert von über 13 und einer Temperatur zwischen 16 und 25 °C durchgeführt wird,
f) die Proteolyse neutralisiert wird, um das erhaltene Proteinhydrolysat zu stabilisieren,
g) das Proteinhydrolysat durch Fest-Flüssig-Trennung gewonnen wird,
h) das Proteinhydrolysat durch Ultrafiltration und/oder Nanofiltration gereinigt wird, die bei einer Trenngrenze zwischen 100 und 25000 Da durchgeführt wird.

2. Verfahren gemäß Anspruch 1, **dadurch gekennzeichnet, dass** die Nanofiltration so durchgeführt wird, dass 3 Banden des Proteinhydrolysats getrennt werden, umfassend eine Bande P1 mit einem Molekulargewicht von weniger als 10000 Da, eine Bande P2 mit einem Molekulargewicht zwischen 10000 und 17000 Da und eine Bande P3 mit einem Molekulargewicht von etwa 23000 Da.

3. Verfahren gemäß einem der Ansprüche 1 und 2, **dadurch gekennzeichnet, dass** die Nanofiltration des Schritts h) mit einer Trenngrenze zwischen 1500 Da und 5000 Da, vorzugsweise mit einer Trenngrenze zwischen 3000 Da und 4500 Da, durchgeführt wird.

4. Verfahren gemäß einem der Ansprüche 1 und 2, **dadurch gekennzeichnet, dass** die Nanofiltration des Schritts h) mit einer Trenngrenze zwischen 10000 Da und 17000 Da durchgeführt wird.

5. Verfahren gemäß einem der Ansprüche 1 und 2, **dadurch gekennzeichnet, dass** die Nanofiltration des Schritts h) mit einer Trenngrenze zwischen 17000 Da und 25000 Da durchgeführt wird.

6. Verfahren gemäß einem der Ansprüche 1 bis 5, **dadurch gekennzeichnet, dass** i) eine Gefriertrocknung des in Schritt h) erhaltenen Proteinhydrolysats durchgeführt wird.

7. Proteinhydrolysat aus dem Presskuchen der ungeschälten und reif gesammelten Samen der Frucht von *Moringa peregrina,* **dadurch gekennzeichnet, dass** dieses eine (Masse/Masse) überwiegende Fraktion P1 aus Derivaten von Aminosäuren, Aminosäuren, Peptiden und Glycopeptiden, deren Molekulargewicht zwischen 1500 Da und 5000 Da liegt, eine Fraktion P2 von etwa 20 % (Masse/Masse) mit einem Molekulargewicht zwischen 10000 und 17000 Da und eine Fraktion P3 von etwa 20 % (Masse/Masse) mit einem Molekulargewicht von etwa 23 000 Da umfasst, wobei dieses durch chemische Proteolyse bei einem pH-Wert von über 13 während einer Dauer von etwa 2 Stunden bei einer Temperatur zwischen 16 und 25 °C erhalten ist und wobei dieses flüssig ist und eine Dichte von über 1 aufweist.

8. Proteinhydrolysat aus dem Presskuchen der ungeschälten und reif gesammelten Samen der Frucht von *Moringa peregrina,* **dadurch gekennzeichnet, dass** dieses die (Masse/Masse) überwiegende Fraktion P1 aus Derivaten von Aminosäurederivaten, Aminosäuren, Peptiden und Glycopeptiden umfasst, deren Molekulargewicht zwischen 1500 Da und 5000 Da liegt, wobei dieses durch chemische Proteolyse bei einem pH-Wert von über 13 während einer Dauer von etwa 2 Stunden bei einer Temperatur zwischen 16 und 25 °C und anschließender Nanofiltration mit einer Trenngrenze zwischen 1500 Da und 5000 Da erhalten ist und wobei dieses flüssig ist und eine Dichte von über 1 aufweist.

9. Proteinhydrolysat aus dem Presskuchen der ungeschälten und reif gesammelten Samen der Frucht von *Moringa peregrina,* **dadurch gekennzeichnet, dass** dieses die Fraktion P2 von etwa 20 % (Masse/Masse) Derivaten von Aminosäuren, Aminosäuren, Peptiden und Glycopeptiden umfasst, deren Molekulargewicht zwischen 10000 Da und 17000 Da liegt, wobei dieses durch chemische Proteolyse bei einem pH-Wert von über 13 während einer Dauer von etwa 2 Stunden bei einer Temperatur zwischen 16 und 25 °C und anschließender Nanofiltration mit einer Trenngrenze zwischen 10000 Da und 17000 Da erhalten ist und wobei dieses flüssig ist und eine Dichte von über 1 aufweist.

10. Proteinhydrolysat aus dem Presskuchen der ungeschälten und reif geernteten Samen der Frucht von *Moringa peregrina,* **dadurch gekennzeichnet, dass** dieses die Fraktion P3 von etwa 20 % (Masse/Masse) Derivaten von Aminosäuren, Aminosäuren, Peptiden und Glycopeptiden mit einem Molekulargewicht von etwa 23000 Da umfasst, wobei dieses durch chemische Proteolyse bei einem pH-Wert von über 13 während einer Dauer von etwa 2 Stunden bei einer Temperatur zwischen 16 und 25 °C und anschließender Nanofiltration mit einer Trenngrenze zwischen 17000 Da und 25000 Da erhalten ist und wobei dieses flüssig ist und eine Dichte von über 1 aufweist.

11. Proteinhydrolysat aus dem Presskuchen der ungeschälten und reif gesammelten Samen der Frucht von *Moringa peregrina,* **dadurch gekennzeichnet, dass** dieses in Kombination das Proteinhydrolysat nach Anspruch 8 und das Proteinhydrolysat nach Anspruch 9 umfasst.

12. Proteinhydrolysat aus dem Presskuchen der Samen von *Moringa peregrina* gemäß einem der Ansprüche 7 bis 11 zur Verwendung als Medikament.

13. Pharmazeutische oder dermatologische Zusammensetzung, **dadurch gekennzeichnet, dass** diese als Wirkstoff eine wirksame Menge eines Proteinhydrolysats aus dem Presskuchen der Samen von *Moringa peregrina* gemäß einem der Ansprüche 7 bis 11 und einen physiologisch verträglichen Trägerstoff umfasst.

14. Pharmazeutische Zusammensetzung gemäß Anspruch 13, **dadurch gekennzeichnet, dass** diese so formuliert ist, dass diese eingenommen werden kann.

15. Dermatologische Zusammensetzung gemäß Anspruch 13, **dadurch gekennzeichnet, dass** diese zur lokalen Anwendung auf der Haut und den Schleimhäuten formuliert ist.

16. Zusammensetzung gemäß Anspruch 13 bis 15, **dadurch gekennzeichnet, dass** das Proteinhydrolysat aus dem Presskuchen der Samen von *Moringa peregrina* in der Zusammensetzung in einer Konzentration von 0,0001 bis 40 Gew.-%, bezogen auf das Gesamtgewicht der Zusammensetzung, umfasst ist.

17. Zusammensetzung gemäß einem der Ansprüche 13 bis 16 zur Verwendung als Medikament zur Behandlung von fibrotischen Erkrankungen und zur Behandlung von Entzündungen, **dadurch gekennzeichnet, dass** diese als Wirkstoff eine wirksame Menge des Proteinhydrolysats nach Anspruch 8 oder 9, vorzugsweise nach Anspruch 9, umfasst.

18. Zusammensetzung gemäß einem der Ansprüche 13 bis 16 zur Verwendung als Medikament zur Behandlung von Krebs, **dadurch gekennzeichnet, dass** diese als Wirkstoff eine wirksame Menge des Proteinhydrolysats nach Anspruch 10 umfasst.

19. Zusammensetzung gemäß einem der Ansprüche 13 bis 16 zur Verwendung als Medikament zur Behandlung von Infektionskrankheiten vom Virustyp zur Hemmung der Spike-COV2-Proteine von SRASCOV2, **dadurch gekennzeichnet, dass** diese als Wirkstoff eine wirksame Menge des Proteinhydrolysats nach Anspruch 7 umfasst.

20. Zusammensetzung gemäß einem der Ansprüche 13 bis 16 zur Verwendung als Medikament zur Behandlung von Hautfibrose und Lentigo, **dadurch gekennzeichnet, dass** diese als Wirkstoff eine wirksame Menge des Proteinhydrolysats gemäß Anspruch 11 umfasst.

## Claims

1. A method for obtaining a protein hydrolysate from *Moringa peregrina* seed cake, **characterized in that** it comprises the following steps, in which:
a) unshelled mature seeds are collected from the ripe *Moringa peregrina* fruit and dried to an internal moisture content of less than 8%,
b) the dried seeds are pressed in a manner such as to separate the oil from the remainder of the seed in a manner such as to obtain the cake comprising less than 6% by weight of residual oil,
c) the cake obtained in step b) is ground,
d) the ground cake obtained in step c) is dispersed in the aqueous phase in a proportion of 90.9/9.1 (mass/mass) respectively,
e) the aqueous dispersion obtained in step d) undergoes chemical proteolysis for a period of approximately 2 hours, at a pH greater than 13 and at a temperature comprised between 16 and 25°C,
f) the proteolysis is neutralised in order to stabilise the protein hydrolysate obtained,
g) the protein hydrolysate is recovered by solid/liquid separation,
h) the protein hydrolysate is purified by ultrafiltration and/or nanofiltration carried out with a cut-off threshold comprised between 100 and 25,000 Da.

2. The method according to claim 1, **characterized in that** the nanofiltration is carried out in a manner such as to separate 3 bands from the protein hydrolysate comprising a P1 band with a molecular weight of less than 10,000 Da, a P2 band with a molecular weight of between 10,000 and 17,000 Da and a P3 band with a molecular weight of about 23,000 Da.

3. The method as claimed in one of claims 1 and 2, **characterised in that** the nanofiltration step h) is carried out with a cut-off threshold comprised between 1,500 Da and 5,000 Da, preferably with a cut-off between 3,000 Da and 4,500 Da.

4. The method as claimed in any one of claims 1 and 2, **characterised in that** the nanofiltration step h) is carried out with a cut-off threshold comprised between 10,000 Da and 17,000 Da.

5. The method as claimed in any one of claims 1 and 2, **characterised in that** the nanofiltration step h) is carried out with a cut-off threshold comprises between 17,000 Da and 25,000 Da.

6. The method as claimed in any one of claims 1 to 5, **characterised in that** i) the protein hydrolysate obtained in step h) is freeze-dried.

7. A protein hydrolysate from seed cake that has not been shelled and has been harvested from ripe *Moringa peregrina* fruit, **characterised in that** it comprises a major fraction P1 (mass/mass) of amino acid derivatives, amino acids, peptides and glycopeptides for which the molecular weight is comprised between 1,500 Da and 5,000 Da, a fraction P2 of approximately 20% (mass/mass) for which the molecular weight is comprised between 10,000 and 17,000 Da, and a fraction P3 of approximately 20% (mass/mass) for which the molecular weight is approximately 23,000 Da, **in that** it is obtained by chemical proteolysis at a pH of more than 13 for a period of approximately 2 hours at a temperature comprised between 16 and 25°C and **in that** it is liquid and has a density of more than 1.

8. A protein hydrolysate from seed cake that has not been shelled and has been harvested from ripe *Moringa peregrina* fruit, **characterised in that** it comprises the major fraction P1 (mass/mass) of amino acid derivatives, amino acids, peptides and glycopeptides for which the molecular weight is comprised between 1,500 Da and 5,000 Da, **in that** it is obtained by chemical proteolysis at pH of more than 13 for a period of approximately 2 hours at a temperature comprised between 16 and 25°C followed by nanofiltration carried out with a cut-off threshold comprised between 1,500 Da and 5,000 Da and **in that** it is liquid and has a density of more than 1.

9. A protein hydrolysate seed cake that has not been shelled and has been harvested from ripe *Moringa peregrina* fruit, **characterised in that** it comprises the P2 fraction of approximately 20% (mass/mass) of amino acid derivatives, amino acids, peptides and glycopeptides for which the molecular weight is comprised between 10,000 Da and 17,000 Da, **in that** it is obtained by chemical proteolysis at pH of more than 13 for a period of approximately 2 hours at a temperature comprised between 16 and 25°C followed by nanofiltration carried out with a cut-off threshold comprised between 10,000 Da and 17,000 Da and **in that** it is liquid and has a density of more than 1.

10. A protein hydrolysate seed cake that has not been shelled and has been harvested from ripe *Moringa peregrina* fruit, **characterised in that** it comprises the P3 fraction of approximately 20% (mass/mass) of amino acid derivatives, amino acids, peptides and glycopeptides for which the molecular weight is approximately 23,000 Da, **in that** it is obtained by chemical proteolysis at pH of more than 13 for a period of about 2 hours at a temperature comprised between 16 and 25°C followed by nanofiltration carried out with a cut-off threshold comprised between 17,000 Da and 25,000 Da and **in that** it is liquid and has a density of more than 1.

11. A protein hydrolysate seed cake that has not been shelled and has been harvested from ripe *Moringa peregrina* fruit, **characterized in that** it comprises in combination the protein hydrolysate of claim 8 and the protein hydrolysate of claim 9.

12. The protein hydrolysate of *Moringa peregrina* seed cake according to any of claims 7 to 11 for use as a medicament.

13. A pharmaceutical or dermatological composition, **characterized in that** it comprises, as an active agent, an effective quantity of a protein hydrolysate of the *Moringa peregrina* seed cake as claimed in one of claims 7 to 11, and a physiologically acceptable excipient.

14. The pharmaceutical composition as claimed in claim 13, **characterised in that** it is formulated for ingestion.

15. The dermatological composition as claimed in claim 13, **characterised in that** it is formulated for local application to the skin and the mucous membranes.

16. The composition as claimed in claims 13 to 15, **characterised in that** the protein hydrolysate of *Moringa peregrina* seed cake is present in the composition in a concentration of 0.0001 to 40% by weight with respect to the total weight of the composition.

17. A composition as claimed in one of claims 13 to 16, for its use as a drug for the treatment of fibrotic diseases and the treatment of inflammation, **characterized in that** it comprises as the active agent, an effective quantity of the protein hydrolysate as claimed in claim 8 or 9, preferably as claimed in claim 9.

18. A composition as claimed in one of claims 13 to 16, for its use as a drug for the treatment of cancer, **characterised in that** it comprises, as the active agent, an effective quantity of the protein hydrolysate as claimed in claim 10.

19. A composition as claimed in one of claims 13 to 16, for its use as a drug for the treatment of infectious diseases of the viral type to inhibit the Spike-COV2 proteins of SARS-COV2, **characterized in that** it comprises, as the active agent, an effective quantity of the protein hydrolysate as claimed in claim 7.

20. A composition as claimed in one of claims 13 to 16, for its use as a drug for the treatment of skin fibrosis and lentigo, **characterized in that** it comprises, as the active agent, an effective quantity of the protein hydrolysate as claimed in claim 11.
